# EUROPEAN PATENT APPLICATION

(11) **EP 3 342 874 A1**
(43) Date of publication of application: **04.07.2018**
(21) Application number: 16839312.2
(22) Date of filing: 24.08.2016
(51) Int. Cl.: C12P 7/22, C12N 1/15, C12N 1/19, C12N 1/21, C12P 7/40, C12P 7/42, C12P 13/22, C12N 15/09

(54) **METHOD FOR PRODUCING AROMATIC COMPOUND AND DERIVATIVE THEREOF**

(30) Priority: 24.08.2015 JP 2015165023
(71) Applicant: RIKEN, Wako-shi, Saitama 351-0198 (JP)
(72) Inventor: NODA Shuhei, Wako-shi Saitama 351-0198 (JP); SHIRAI Tomokazu, Wako-shi Saitama 351-0198 (JP)
(74) Representative: J A Kemp
(86) International application number: PCT/JP2016/074649
(87) International publication number: WO 2017/033965

(57) **Abstract**

The present invention provides a method for producing an aromatic compound such as salicylic acid and a derivative thereof with high productivity using a microorganism. The present invention provides: a method for producing a microorganism having a sugar metabolic pathway modified, including suppressing the expression of a gene encoding a phosphotransferase system enzyme of the microorganism, suppressing the expression of a gene encoding pyruvate kinase of the microorganism, and introducing, into the microorganism, one or more genes encoding an enzyme that enables the microorganism to synthesize an aromatic compound from chorismic acid or isochorismic acid; a modified microorganism obtained by the method; and a method for producing an aromatic compound and a derivative thereof, including culturing the microorganism, and recovering an aromatic compound or the like from the culture.

SEQUENCE LISTING

<110>  RIKEN

<120>  Method for Producing an Aromatic Compound and a Derivative thereof

<130>  PH-6674-PCT

<150>  JP 2015-165023
<151>  2015-08-24

<160>  51

<170>  PatentIn version 3.5

<210>  1
<211>  53
<212>  DNA
<213>  Artificial

<220>
<223>  primer

<400>  1
gtcgacggta tcgataaaga ggagaaaaag cttatgaaaa cccctgaaga ttg          53


<210>  2
<211>  56
<212>  DNA
<213>  Artificial

<220>
<223>  primer

<400>  2
caggaattcg atatctaaat gatgatgatg atgatgggct gcaccacggg tctgac       56


<210>  3
<211>  41
<212>  DNA
<213>  Artificial

<220>
<223>  primer

<400>  3
attaaagagg agaaaggtac catgcaatca cttactacgg c                       41


<210>  4
<211>  62
<212>  DNA
<213>  Artificial

<220>
<223>  primer

<400>  4
ctcgaggggg ggcccttact tgtcatcgtc atccttgtag tcttccattt gtaataaagt   60

ac                                                                  62

<210>  5
<211>  41
<212>  DNA
<213>  Artificial

<220>
<223>  primer

<400>  5
attaaagagg agaaaggtac catggatacg tcactggctg a                41


<210>  6
<211>  62
<212>  DNA
<213>  Artificial

<220>
<223>  primer

<400>  6
ctcgaggggg ggcccttact tgtcatcgtc atccttgtag tcatgcaatc caaaaacgtt      60

ca                                                                      62


<210>  7
<211>  41
<212>  DNA
<213>  Artificial

<220>
<223>  primer

<400>  7
attaaagagg agaaaggtac catgagccgt ctggcaccgc t                41


<210>  8
<211>  62
<212>  DNA
<213>  Artificial

<220>
<223>  primer

<400>  8
ctcgaggggg ggcccttact tgtcatcgtc atccttgtag tcggcaacac cacgctgcag      60

ag                                                                      62


<210>  9
<211>  35
<212>  DNA
<213>  Artificial

<220>
<223>  primer

<400>  9

gacgatgacg ataagatgtc acaccccgcg ttaac 35

<210> 10
<211> 38
<212> DNA
<213> Artificial

<220>
<223> primer

<400> 10
tgcagatctc gagctttagt acaacggtga cgccggta 38

<210> 11
<211> 51
<212> DNA
<213> Artificial

<220>
<223> primer

<400> 11
attaaagagg agaaaggtac catgatcctg cttatagata actacgattc t 51

<210> 12
<211> 47
<212> DNA
<213> Artificial

<220>
<223> primer

<400> 12
ttacttgtca tcgtcatcct tgtagtcgcg atgcaggaaa ttagcca 47

<210> 13
<211> 63
<212> DNA
<213> Artificial

<220>
<223> primer

<400> 13
gacgatgaca agtaaaaaga ggagaaactc gagatgaaga cgttatctcc cgctgtgatt 60

act 63

<210> 14
<211> 66
<212> DNA
<213> Artificial

<220>
<223> primer

<400> 14
ctcgaggggg ggcccttaat gatgatgatg atgatgcttc tccagttgct tcaggatacg 60

attaac                                                                  66

<210>  15
<211>  35
<212>  DNA
<213>  Artificial

<220>
<223>  primer

<400>  15
attaaagagg agaaaatgca aacacaaaaa ccgac                                  35

<210>  16
<211>  35
<212>  DNA
<213>  Artificial

<220>
<223>  primer

<400>  16
ctcgaggggg ggcccttaca gaatcggttg cagcg                                  35

<210>  17
<211>  40
<212>  DNA
<213>  Artificial

<220>
<223>  primer

<400>  17
catcatcatc atggtatgag aaactatcct gcagaacctt                             40

<210>  18
<211>  40
<212>  DNA
<213>  Artificial

<220>
<223>  primer

<400>  18
tctcgagctc ggatcttacg ctttcggttc aaaacgcgcc                             40

<210>  19
<211>  35
<212>  DNA
<213>  Artificial

<220>
<223>  primer

<400>  19
catcatcatc atggtatgct gaatccgagc agcct                                  35

<210>  20
<211>  35
<212>  DNA
<213>  Artificial

<220>
<223>  primer

<400>  20
tctcgagctc ggatcttaca tcacaacacc ttcaa                                35


<210>  21
<211>  24
<212>  DNA
<213>  Artificial

<220>
<223>  primer

<400>  21
accatgatga tgatgatgag aacc                                            24


<210>  22
<211>  24
<212>  DNA
<213>  Artificial

<220>
<223>  primer

<400>  22
gatccgagct cgagatctgc agct                                            24


<210>  23
<211>  40
<212>  DNA
<213>  Artificial

<220>
<223>  primer

<400>  23
caggacgcac tgaccatgac aaagtatgca ttagtcggtg                          40


<210>  24
<211>  61
<212>  DNA
<213>  Artificial

<220>
<223>  primer

<400>  24
tctttatcga taccgtcgac ttaatgatga tgatgatgat gcagaatgtg acctaaggtc    60

t                                                                     61

<210>  25
<211>  53
<212>  DNA
<213>  Artificial

<220>
<223>  primer

<400>  25
gtcgacggta tcgataaaga ggagaaaaag cttatgcctg acgctaaaaa aca          53


<210>  26
<211>  62
<212>  DNA
<213>  Artificial

<220>
<223>  primer

<400>  26
tgcctctagc acgcgttact tgtcatcgtc atccttgtag tcatcgtgag cgcctatttc   60

gc                                                                  62


<210>  27
<211>  75
<212>  DNA
<213>  Artificial

<220>
<223>  primer

<400>  27
atgttccagc aagaagttac cattaccgct ccgaacggtc tgcacacccg cctaggtcta   60

gggcggcgga tttgt                                                    75


<210>  28
<211>  75
<212>  DNA
<213>  Artificial

<220>
<223>  primer

<400>  28
ttagcagatt gtttttcttt caatgaactt gttaaccagc gtcattaact taatacgact   60

cactataggg ctcga                                                    75


<210>  29
<211>  72
<212>  DNA
<213>  Artificial

<220>
<223>  primer

<400>  29

atgaaaaaga ccaaaattgt ttgcaccatc ggaccgaaaa ccgaatctga aattaaccct 60

cactaaaggg cg 72


<210> 30
<211> 73
<212> DNA
<213> Artificial

<220>
<223> primer

<400> 30
ttacaggacg tgaacagatg cggtgttagt agtgccgctc ggtaccagtg taatacgact 60

cactataggg ctc 73


<210> 31
<211> 72
<212> DNA
<213> Artificial

<220>
<223> primer

<400> 31
atgtccagaa ggcttcgcag aacaaaaatc gttaccacgt taggcccagc aattaaccct 60

cactaaaggg cg 72


<210> 32
<211> 73
<212> DNA
<213> Artificial

<220>
<223> primer

<400> 32
ttactctacc gttaaaatac gcgtggtatt agtagaaccc acggtactca taatacgact 60

cactataggg ctc 73


<210> 33
<211> 72
<212> DNA
<213> Artificial

<220>
<223> primer

<400> 33
atgacatcgg aaaacccgtt actggcgctg cgagagaaaa tcagcgcgct aattaaccct 60

cactaaaggg cg 72


<210> 34
<211> 73

<212>  DNA
<213>  Artificial

<220>
<223>  primer

<400>  34
tcaggttgga tcaacaggca ctacgttctc acttgggtaa cagcccaata taatacgact      60

cactataggg ctc                                                         73


<210>  35
<211>  73
<212>  DNA
<213>  Artificial

<220>
<223>  primer

<400>  35
agccgctttg gactgcctat ttatgttccg gagcgcgagg catctatgtt taatacgact      60

cactataggg ctc                                                         73


<210>  36
<211>  1887
<212>  DNA
<213>  Artificial

<220>
<223>  term-PAlacO-1-FRT-gb2-neo-FRT in pCFTdeltain

<400>  36
gctagcccta ggtctagggc ggcggatttg tcctactcag gagagcgttc accgacaaac      60

aacagataaa acgaaaggcc cagtctttcg actgagcctt tcgttttatt tgatgcctct     120

agcacgcggg atcctttatc atcgtcgtct ttatagtcga tatcaagctt ggtcagtgcg     180

tcctgctgat gtgctcagta tcttgttatc cgctcacaat gtcaattgtt atccgctcac     240

aattaattaa ccctcactaa agggcggccg cgaagttcct attctctaga aagtatagga     300

acttcattct accgggtagg ggaggcgctt ttcccaaggc agtctggagc atgcgcttta     360

gcagccccgc tgggcacttg gcgctacaca agtggcctct ggcctcgcac acattccaca     420

tccaccggta ggcgccaacc ggctccgttc tttggtggcc ccttcgcgcc accttccact     480

cctcccctag tcaggaagtt cccccccgcc ccgcagctcg cgtcgtgcag gacgtgacaa     540

atggaagtag cacgtctcac tagtctcgtg cagatggaca gcaccgctga gcaatggaag     600

cgggtaggcc tttggggcag cggccaatag cagctttgct ccttcgcttt ctgggctcag     660

aggctgggaa ggggtgggtc cgggggcggg ctcaggggcg ggctcagggg cggggcgggc     720

gcccgaaggt cctccggagg cccggcattc tgcacgcttc aaaagcgcac gtctgccgcg     780

ctgttctcct cttcctcatc tccgggcctt tcgacctgca gcagcacgtg ttgacaatta     840

atcatcggca tagtatatcg gcatagtata atacgacaag gtgaggaact aaaccatggg 900

atcggccatt gaacaagatg gattgcacgc aggttctccg gccgcttggg tggagaggct 960

attcggctat gactgggcac aacagacgat cggctgctct gatgccgccg tgttccggct 1020

gtcagcgcag gggcgcccgg ttctttttgt caagaccgac ctgtccggtg ccctgaatga 1080

actgcaggac gaggcagcgc ggctatcgtg gctggccacg acgggcgttc cttgcgcagc 1140

tgtgctcgac gttgtcactg aagcgggaag ggactggctg ctattgggcg aagtgccggg 1200

gcaggatctc ctgtcatctc accttgctcc tgccgagaaa gtatccatca tggctgatgc 1260

aatgcggcgg ctgcatacgc ttgatccggc tacctgccca ttcgaccacc aagcgaaaca 1320

tcgcatcgag cgagcacgta ctcggatgga agccggtctt gtcgatcagg atgatctgga 1380

cgaagagcat caggggctcg cgccagccga actgttcgcc aggctcaagg cgcgcatgcc 1440

cgacggcgag gatctcgtcg tgacccatgg cgatgcctgc ttgccgaata tcatggtgga 1500

aaatggccgc ttttctggat tcatcgactg tggccggctg ggtgtggcgg accgctatca 1560

ggacatagcg ttggctaccc gtgatattgc tgaagagctt ggcggcgaat gggctgaccg 1620

cttcctcgtg ctttacggta tcgccgctcc cgattcgcag cgcatcgcct tctatcgcct 1680

tcttgacgag ttcttctgag cgggactctg gggttcgaat aaagaccgac caagcgacgt 1740

ctgagagctc cctggcgaat tcggtaccaa taaaagagct ttattttcat gatctgtgtg 1800

ttggtttttg tgtgcggcgc ggaagttcct attctctaga aagtatagga acttcctcga 1860

gccctatagt gagtcgtatt agtcgac 1887

<210> 37
<211> 1431
<212> DNA
<213> Pseudomonas aeruginosa

<400> 37
atgagccgtc tggcaccgct gagccagtgt ctgcatgcac tgcgtggcac ctttgaacgt 60

gcaattggtc aggcacaggc actggatcgt ccggttctgg ttgcagcaag ctttgaaatt 120

gatccgctgg atcctctgca ggtttttggt gcatgggatg atcgtcagac cccgtgtctg 180

tattgggaac agccggaact ggcatttttt gcatggggtt gtgcactgga actgcagggt 240

catggtgaac agcgttttgc acgtattgaa gaaaattggc agctgctgtg tgcagatgca 300

gttgttgaag gtccgctggc tccgcgtctg tgtggtggtt ttcgttttga tccgcgtggt 360

ccgcgtgaag aacactggca ggcatttgca gatgccagcc tgatgctggc aggtattacc 420

gttctgcgtg aaggtgaacg ttatcgtgtt ctgtgtcagc atctggcaaa accgggtgaa 480

gatgcactgg cactggcagc atatcattgt agcgcactgc tgcgtctgcg tcagcctgca 540

cgtcgtcgtc cgagtggtcc gacagccggt gcacagggtg atgcaagcgc acaagaacgt 600

cgtcagtggg aagcaaaagt tagtgatgca gttagcagcg ttcgtcaggg tcgttttggt 660

aaagttgttc tggcacgtac ccaggcacgt ccgctgggtg atattgaacc gtggcaggtt 720

attgaacatc tgcgtctgca gcatgccgat gcacagctgt ttgcatgtcg tcgtggtaat 780

gcatgttttc tgggtgcaag tccggaacgt ctggttcgta ttcgtgccgg tgaagcactg 840

acccatgcac tggcaggcac cattgcacgt ggtggtgatg cacaagaaga tgcacgtctg 900

ggtcaggcac tgctggatag cgcaaaagat cgtcatgaac atcagctggt tgttgaagca 960

attcgtaccg cactggaacc gtttagcgaa gttctggaaa ttccggatgc accgggtctg 1020

aaacgtctgg cacgtgttca gcatctgaat accccgattc gtgcccgtct ggcagatgcc 1080

ggtggtattc tgcgcctgct gcaggccctg catccgacac cggcagttgg tggttatccg 1140

cgtagcgcag cactggatta tattcgtcag catgaaggta tggatcgtgg ttggtatgca 1200

gcaccgctgg gttggctgga tggtgaaggt aatggtgatt ttctggttgc actgcgtagt 1260

gcactgctga caccgggtcg tggttacctg tttgcaggtt gtggtctggt tggtgatagc 1320

gaaccggcac atgaatatcg tgaaacctgt ctgaaactga gcgcaatgcg tgaagccctg 1380

agcgccattg gtggtctgga tgaagttcct ctgcagcgtg gtgttgccta a 1431

<210> 38
<211> 306
<212> DNA
<213> Pseudomonas aeruginosa

<400> 38
atgaaaaccc ctgaagattg taccggtctg gcagatattc gtgaagcaat tgatcgtatt 60

gatctggata ttgttcaggc actgggtcgt cgtatggatt atgttaaagc agcaagccgt 120

tttaaagcaa gcgaagcagc aattccggca ccggaacgtg ttgcagcaat gctgccggaa 180

cgcgcacgtt gggctgaaga aaatggtctg gatgcaccgt ttgttgaagg tctgtttgca 240

cagattatcc attggtatat tgccgagcag atcaaatatt ggcgtcagac ccgtggtgca 300

gcctaa 306

<210> 39
<211> 498
<212> DNA
<213> Escherichia coli

<400> 39
atgtcacacc ccgcgttaac gcaactgcgt gcgctgcgct attgtaaaga gatccctgcc 60

ctggatccgc aactgctcga ctggctgttg ctggaggatt ccatgacaaa acgttttgaa 120

cagcagggaa aaacggtaag cgtgacgatg atccgcgaag ggtttgtcga gcagaatgaa 180

atccccgaag aactgccgct gctgccgaaa gagtctcgtt actggttacg tgaaattttg 240

ttatgtgccg atggtgaacc gtggcttgcc ggtcgtaccg tcgttcctgt gtcaacgtta 300

agcgggccgg agctggcgtt acaaaaattg ggtaaaacgc cgttaggacg ctatctgttc 360

acatcatcga cattaacccg ggactttatt gagataggcc gtgatgccgg gctgtggggg 420

cgacgttccc gcctgcgatt aagcggtaaa ccgctgttgc taacagaact gtttttaccg 480

gcgtcaccgt tgtactaa 498

<210> 40
<211> 1023
<212> DNA
<213> Streptomyces hygroscopicus

<400> 40
atgctgaatc cgagcagcct ggttctgaat ggtctgacca gctattttga aaatggtcgt 60

gcacgtgttg ttccgcctgt tggtcgtaat attctgggtg ttgttaatta tgccagcgtt 120

tgtgaatatc cgaccctgga tcatggttat ccggaactgg aaattaacat ggttgcaccg 180

accgcagaac cgtttgccga agtttgggtt accgatgcag aaagcgaaca tggtgaacgt 240

gatggtatta cctatgcaca tgatggcgaa tatttctttt gtgcaggtcg tgttccgcca 300

accggtcgtt ataccgaagc aacccgtgca gcatatgtta ccatgtttga actgctggaa 360

gaatttggtt atagcagcgt ttttcgcatg tggaacttta ttggtgatat caatcgtgat 420

aatgccgaag gcatggaagt gtatcgtgat ttttgtcgtg gtcgtgccga agcatttgaa 480

cagtgtcgtc tggaatttga tcagtttccg gcagcaaccg gtattggtag ccgtggtggt 540

ggtattgcat tttatctgct ggcatgtcgt agcggtggtc atgttcatat tgaaaatccg 600

cgtcaggttc cggcatatca ttatccgaaa cgttatggtc cgcgtgcacc gcgttttgca 660

cgtgccacct atctgccgag ccgtgcagcc gatggtgttg gtggtcaggt ttttgttagc 720

ggcaccgcaa gcgttctggg tcatgaaacc gcacatgaag gtgatctggt taaacaatgc 780

cgtctggcac tggaaaatat tgaactggtt attagtggtg gtaatctggc agcacatggt 840

attagcgcag gtcatggcct gaccgcactg cgtaacatta aagtttatgt tcgtcgtagc 900

gaagatgttc cggcagttcg tgaaatttgt cgtgaagcat ttagtccgga tgccgatatt 960

gtttatctga ccgttgatgt ttgccgtagc gatctgctgg ttgaaattga aggtgttgtg 1020

atg 1023

<210> 41
<211> 1023
<212> DNA
<213> Streptomyces sp.

<400> 41
atgaatccga gcagcctggt tctgaatggt ctgaccagct attttgaaaa tggtcgtgcc 60

ggtggtgttc cgcctgcagg tcgtaatatt ctgggtgttg ttaattatgc cagcgtttgt 120

gaatatccga ccctggatca tggttatccg gaactggcca ttaatatggt tgcaccgacc 180

gcagaaccgt ttgccgaagt ttgggttacc gatgccgaag cagaacatgg tgaacgtgat 240

ggtattacct atgcacatga tggcgaatat ttcttttgtg cgggtcgtgt tcctccgacg 300

ggtcgttata ccgaagcaac ccgtgcagca tatgttacca tgtttgaact gctggaagaa 360

tttggttata gcagcgtttt tcgcatgtgg aactttattg gtgatatcaa tcgtgataat 420

gccgaaggca tggaagtgta tcgtgatttt tgtcgtggtc gtgcggaagc atttgaacag 480

tgtcgtctgg aatttgatca gtttccggca gcaaccggta ttggtagccg tggtggtggt 540

attgcatttt atctgctggc atgtcgtagc ggtggtcatg ttcatattga aaatccgcgt 600

caggttccgg catatcatta tccgaaacgt tatggtccgc gtgcaccgcg ttttgcacgt 660

gccacctatc tgccgagccg tgcagccgat ggcggtggtg gtcaggtttt tgttagcggc 720

accgcaagcg ttctgggtca tgaaaccgca catgaaggtg atctggttaa acaatgccgt 780

ctggcactgg aaaatattga actggttatt agtggtggta atctggcagc acatggtatt 840

agcgcaggtc atggcctgac cgcactgcgt aacattaaag tttatgttcg tcgtgcagaa 900

gatgttccgg cagttcgtga aatttgtcgt gaagcattta gtccggatgc cgatattgtt 960

tatctgaccg ttgatgtttg ccgtagcgat ctgctggttg aaattgaagg tgttgtgatg 1020

taa 1023

<210> 42
<211> 888
<212> DNA
<213> Escherichia coli

<400> 42
cttgatatcg aattcaaaga ggagaaactg cagatgtttc tgatcaacgg tcataaacaa 60

gaaagcctgg cagttagcga tcgtgcaacc cagtttggtg atggttgttt taccaccgca 120

cgtgttattg atggtaaagt tagcctgctg agcgcacata ttcagcgtct gcaggatgca 180

tgccagcgtc tgatgattag ctgtgatttt tggcctcagc tggaacaaga aatgaaaacc 240

ctggcagcag aacagcagaa tggtgttctg aaagttgtta ttagccgtgg tagcggtggt 300

cgtggttata gcaccctgaa tagcggtccg gcaacccgta ttctgagcgt taccgcatat 360

ccggcacatt acgatcgtct gcgtaatgaa ggtattaccc tggcactgag tccggttcgt 420

ctgggtcgta atccgcatct ggcaggtatt aaacatctga atcgtctgga acaggttctg 480

attcgtagcc acctggaaca gaccaatgca gatgaagcac tggttctgga tagcgaaggt 540

tgggttaccg aatgttgtgc agcaaacctg ttttggcgta aaggtaatgt tgtttataca 600

ccgcgtctgg atcaggcagg cgttaatggt attatgcgtc agttttgtat tcgtctgctg 660

gcacagagca gctatcagct ggttgaagtt caggcaagcc tggaagaaag tctgcaggca 720

gatgaaatgg ttatttgtaa tgcactgatg ccggttatgc cggtgtgtgc atgtggtgat 780

gttagcttta gcagcgcaac cctgtatgaa tatctggcac cgctgtgtga acgtccgaat 840

gaacagaaac tgattagcga agaagatctg taactgcagc ccgggga 888

<210> 43
<211> 1158
<212> DNA
<213> Escherichia coli

<400> 43
attaaagagg agaaaggtac catggttgct gaattgaccg cattacgcga tcaaattgat 60

gaagtcgata aagcgctgct gaatttatta gcgaagcgtc tggaactggt tgctgaagtg 120

ggcgaggtga aaagccgctt tggactgcct atttatgttc cggagcgcga ggcatctata 180

ttggcctcgc gtcgtgcaga ggcggaagct ctgggtgtac cgccagatct gattgaggat 240

gttttgcgtc gggtgatgcg tgaatcttac tccagtgaaa acgacaaagg atttaaaaca 300

ctttgtccgt cactgcgtcc ggtggttatc gtcggcggtg gcggtcagat gggacgcctg 360

ttcgagaaga tgctgaccct ctcgggttat caggtgcgga ttctggagca acatgactgg 420

gatcgagcgg ctgatattgt tgccgatgcc ggaatggtga ttgttagtgt gccaatccac 480

gttactgagc aagttattgg caaattaccg cctttaccga aagattgtat tctggtcgat 540

ctggcatcag tgaaaaatgg gccattacag gccatgctgg tggcgcatga tggtccggtg 600

ctggggctac acccgatgtt cggtccggac agcggtagcc tggcaaagca agttgtggtc 660

tggtgtgatg gacgtaaacc ggaagcatac caatggtttc tggagcaaat tcaggtctgg 720

ggcgctcggc tgcatcgtat tagcgccgtc gagcacgatc agaatatggc gtttattcag 780

gcactgcgcc actttgctac ttttgcttac gggctgcacc tggcagaaga aaatgttcag 840

cttgagcaac ttctggcgct ctcttcgccg atttaccgcc ttgagctggc gatggtcggg 900

cgactgtttg ctcaggatcc gcagctttat gccgacatca ttatgtcgtc agagcgtaat 960

ctggcgttaa tcaaacgtta ctataagcgt ttcggcgagg cgattgagtt gctggagcag 1020

ggcgataagc aggcgtttat tgacagtttc cgcaaggtgg agcactggtt cggcgattac 1080

gttcagcgtt ttcagagtga aagccgcgtg ttattgcgtc aggcgaatga caatcgccag 1140

taagggcccc ccctcgag 1158

<210> 44
<211> 1413
<212> DNA
<213> Pasteurella multocida

<400> 44
attaaagagg agaaaggtac catgagaaac tatcctgcag aaccttataa agtcaaagct 60

gttgaaccga ttgcaatgac gactcgtgag caacgtgaag cctacatgaa aaaagcaggc 120

tacaatacgt ttttactcaa ttcagaagaa gtgtatattg atttattaac cgatagtggt 180

acctcagcca tgagtgataa gcaatgggcg ggtcttatga tcggtgatga agcttatgca 240

ggtagccgta acttcatgca tttacaagac gtggtcagag aatattatgg tttcaaatat 300

gtcgtaccaa ctcaccaagg tcgtggtgca gaaaacttac tttcaaccat tatgatcaaa 360

ccgggcgatt atgtaccggg taatatgtat tttactacaa cacgcgcgca ccaagagcgt 420

aatggggcga ctttcgttga tattattatt gatgaagccc atgattcaca aattgattta 480

cctttcaaag gcaatgtcga tgtgaagaaa ttacaaaaac tgattgatga agtgggtgcg 540

gataaaattc cttacatctg tttagcggtt accgtaaact tagcgggtgg acaacctgtg 600

agtatggcaa acatgcgtga agtaaaagca ttatgtagca aacatggtat taaagtgatg 660

tttgatgcca cacgttgtgt agaaaatgcg tacttcatta aagagcgtga agcagagtat 720

aaagatgcaa ccatcaaaga tattctaaaa gaaatgatga gttatgcaga tggttgtacc 780

atgagtggta aaaaagactg tcttgtgaat attggtggtt tcttatgtat taatgatgat 840

gatctttatc aacaagcttg tgaactcgtg gtgctctttg aagggatgcc atcttatggt 900

ggactagctg gtcgtgatat ggaagcaatg gcaattggta ttacggaatc tgtcgatttc 960

cactatatcc agcaccgtgt tgcacaatgt tactacctcg cggataaatt agaagcggcg 1020

ggtgtgccga ttgtgaaacc agtgggaggt catgcggtat tcttagatgc gaagaaattc 1080

cttccacata ttccacaaga acaattccca gcacagatgc ttgctgcgca aatttatatt 1140

gagggtggcg tacgttcaat ggaacgtggt attgtatcgg cgggtcgtga taagaaaacc 1200

ggtgcaaacc atacgcctaa attagaatta gtgcgtttaa ctatcccacg tcgtgtttat 1260

acttatgcgc atttagatca tgttgctgac accattatta aactctttaa acacagagat 1320

gacattaaag gtcttgatat ggtgtatgag cctaaattat tacgtttctt tacggcgcgt 1380

tttgaaccga aagcgtaagg gccccccctc gag 1413

<210> 45
<211> 1326
<212> DNA
<213> Pseudomonas putida TK2440

<400> 45
attaaagagg agaaaggtac catgcagaac agtaccagcg ccctgaacgt tagcatcatt 60

ggcggcggta tcgcaggcgt tgcactggcc ctggacttat gtcgccacgc ccacctgaac 120

gtgcagctgt tcgaggcagc cccggccttt ggcgaagttg gtgccggtgt tagcttcggc 180

gccaatgcag tgcgtgcaat cgccggtctg ggtatcgcag agccgtacgg caaaattgcc 240

gacagtaatc cggccccgtg gcaggacatc tggttcgaat ggcgcaatgg ccgtgatgcc 300

aaatacctgg gttgcagcgt tgccgaaggc gttggtcaaa gcagtgtgca ccgtgccgat 360

ttcctggacg ctctggcttc tcagctgccg gacggtatcg ctcagttcgg taaacgtgct 420

cagcgtgttg aacaggacgg tgagcaagtg cgtgtgacat tcacagacgg cagcgagcac 480

cgctgcgatc tgctgattgg tgccgacggt atcaagagta gcatccgtga ccacgtgtta 540

cagggcctga atcaaccgct ggcaagcccg cgttttagcg gcacctgcgc ctatcgcggt 600

ctgatcgata gccagcagct gcgtgaggcc tatcgtgccc gtggcgtgga cgagcatctg 660

attgacgtgc cgcagatgta cctgggcctg gacggccaca tcctgacctt cccggttaaa 720

caaggccgcc tgatcaacgt ggtggccttc atcagtgacc gcagccaacc gaacccggtt 780

tggccgagcg atacaccgtg ggttcgtaat gccacccaag ccgagatgct ggccgcattc 840

gagggctggg atgatgcagc ccaagtgctg ctggagtgca tcccgacccc tagtctgtgg 900

gccctgcacg acctggcaga attaccgggc tacgttcacg gccgtgttgg cttaatcggc 960

gacgccgccc acgcaatgtt accgcatcag ggtgccggtg caggtcaggg tctggaggat 1020

gcctggttac tggcccgcct gctggaagac ccgaaggtgc tggacaaacg cccgcaggca 1080

gtgctggatg cctatgacgc cgttcgtcgt cctcgtgcct gccgtgtgca gcgtaccagc 1140

ttcgaggccg gcgaactgta tgagttccgt gacccggccg tgttagccga cgaggagcgt 1200

ctgggcaaag ttctggccga acgctttgac tggctgtgga accacgacat gcaggaagac 1260

ttattacagg cccgtgagct gctgggttta cgtgcccaag ccgcctaata ggggcccccc 1320

ctcgag 1326

<210> 46
<211> 987
<212> DNA
<213> Pseudomonas putida DOT-T1E

<400> 46
gtcgacggta tcgatcatta aagaggagaa aggatgaccg tgaaaattag ccataccgca 60

gatattcagg ccttttttaa ccgtgttgca ggtctggatc atgcagaagg taatccgcgt 120

tttaaacaaa ttattctgcg tgttctgcag gataccgcac gtctgattga agatctggaa 180

attaccgaag atgaattttg gcatgccgtg gattatctga atcgtctggg tggtcgtaat 240

gaagcaggtc tgctggcagc cggtctgggt attgaacatt ttctggatct gctgcaggat 300

gcaaaagatg ccgaagcagg cctgggtggt ggtacaccgc gtaccattga aggtccgctg 360

tatgttgcgg gtgcaccgct ggcacagggt gaagcacgta tggatgatgg caccgatccg 420

ggtgttgtta tgtttctgca gggtcaggtt tttgatgcag atggcaaacc tctggcaggc 480

gcaaccgttg atctgtggca tgcaaatacc cagggcacct atagctattt tgatagcacc 540

cagagcgaat ttaatctgcg tcgtcgtatt attaccgatg cggaaggtcg ttatcgtgca 600

cgtagcattg ttccgagcgg ttatggttgt gatccgcagg gtccgaccca agaatgtctg 660

gacctgctgg gtcgtcatgg tcagcgtccg gcacatgttc attttttcat tagcgcaccg 720

ggtcatcgtc atctgaccac acagattaac tttgccggtg ataaatatct gtgggatgat 780

tttgcatatg ccacccgtga tggtctgatt ggtgaactgc gttttgttga agatgcagca 840

gcagcacgtg atcgtggtgt tcagggtgaa cgttttgcag aactgagctt tgattttcgc 900

ctgcagggtg ccaaaagtcc ggatgcagaa gcccgtagcc atcgtccgcg tgcactgcaa 960

gaaggttaat aggatatcga attcctg 987

<210> 47
<211> 1200
<212> DNA
<213> Rhodococcus jostii RHA1

<400> 47
atgagcaatc tgcaggatgc ccgtattatc attgccggtg gtggtattgg tggtgcagca 60

aatgcactgg cactggccca gaaaggtgca aatgttaccc tgtttgaacg tgcaagcgaa 120

tttggtgaag ttggtgcagg tctgcaggtt ggtccgcatg gtgcacgtat tctggatagc 180

tggggtgttc tggatgatgt tctgagccgt gcatttctgc cgaaaaatat cgtttttcgt 240

gatgccatta ccgcagaagt tctgaccaaa attgatctgg gtagcgaatt tcgtggtcgt 300

tatggtggtc cgtattttgt tacccatcgt agcgatctgc atgcaaccct ggttgatgca 360

gcacgtgcag ccggtgcaga actgcatacc ggtgttaccg ttaccgatgt tattaccgaa 420

ggtgataaag caattgtgag caccgatgat ggtcgtaccc atgaagcaga tattgcactg 480

ggtatggatg gtctgaaaag ccgtctgcgt gaaaaaatca gcggtgatga accggttagc 540

agcggttatg cagcatatcg tggcaccacc ccgtatcgtg atgttgaact ggatgaagat 600

attgaagatg tggtgggtta tattggtccg cgttgtcatt ttattcagta tccgctgcgt 660

ggtggtgaaa tgctgaatca ggttgcagtt tttgaaagtc cgggattcaa aaacggcatt 720

gaaaattggg gtggtccgga agaactggaa caggcatatg cacattgcca tgaaaatgtt 780

cgtcgcggta ttgattatct gtggaaagat cgttggtggc cgatgtatga tcgtgaaccg 840

attgaaaact gggttgatgg tcgcatgatt ctgctgggtg atgccgcaca tccgcctctg 900

cagtatctgg caagcggtgc agttatggca atcgaagatg caaaatgtct ggcagattat 960

gcagccgaag attttagcac cggtggtaat agcgcatggc ctcagattct gaaagaagtt 1020

aataccgaac gcgcaccgcg ttgtaatcgt attctgacca ccggtcgtat gtggggtgaa 1080

ctgtggcatc tggatggcac cgcacgtatt gcacgtaatg aactgtttcg tacccgtgat 1140

accagcagct ataaatacac cgattggctg tggggttata gcagcgatcg tgcaagctaa 1200

<210> 48
<211> 1134

<212>  DNA
<213>  Rhodococcus sp. NCIMB 12038

<400>  48
attaaagagg agaaaggtac catgaccgca accgaaccgc atgttgatga tgatccggca      60

ctgaaacagc tgtataccga ttttgaagca gaacatctga atccgctgtg gacacagctg     120

ggtgatctga tgccgatgac cccgaccagc cgtgcagttc cgtttgtttg gaaatggtca     180

accctgtatc cgctggcaca gcgtgccggt gatctggttc cggttggtcg tggtggtgaa     240

cgtcgtgcaa ttgccctggc aaatccgggt ctgggtggtg ttccgtatgt taccccgacc     300

ctgtgggcag caattcagta tctgggtccg aaagaagttg caccggaaca tcgtcatgca     360

cagaatgcat ttcgttttgt tgttgaaggt gaaggtgttt ggaccgttgt taatggtgat     420

ccggttgcaa tgcgtcgtgg tgattttctg ctgacaccgg gttggcattt tcatggtcat     480

cataatgaaa ccgatcagcc gatggcatgg attgatggtc tggatatccc gtttgttcat     540

tataccgata ccggcttttt tgaatttggc agcgaaaatg ttaccgatga tagcacaccg     600

gatgttagcc gtagcgaacg tctgtgggca catcctggtc tgcgtccgct ggttggcctg     660

gatgcaaaaa ccagcagccc gattgcagca tatcgttggg aacacaccga tgcagcactg     720

cgtgaacagc tggcactgga agatgaaggt tatgcagcaa ccaccgaacc gggtcatgca     780

gcagttcgtt ataccaatcc gaccacaggt ggtgatgtta tgccgaccat tcgtgcagaa     840

tttcatcgtc tgcgtgcggg tgcacatacc cgtccgcgtc gtgatgttgg tagcaccgtt     900

tatcaggttt ttgaaggtag cggtcgtttt gtgctgggtg gtcagacccg taccgtgggt     960

aaaggtgata tgattgttgt tccgagctgg accgaatggt caattgaagc cgataccgaa    1020

tttgacctgt ttgcatttag tgatgcaccg attgttgaac gtctgcattt tcaccgtacc    1080

tatattagcg aaggtgcaca tcatcatcat catcattaag ggccccccct cgag          1134


<210>  49
<211>  939
<212>  DNA
<213>  Pseudomonas alcaligenes NCIMB 9867

<400>  49
gtcgacggta tcgatttcat taaagaggag aaaggtatga aaatctgccg ctttaatgaa      60

aatcgtctgg gtgttgttga tggtgatgaa gttctggatg ttaccgaagc actgagcgtt     120

ctgccgagct atgaatatcc gctgcctggt tatgatccgc tgattaaaca tctggatgca     180

ctgctggcac gtattgaaac cctgattaaa gatgcaccgc gtattctgct ggcagatgtt     240

cgtctgctga gtccggttgc aaatccgggt aaaatcattg cagcaccgat taactatacc     300

cgtcatctgc aagaagtgct ggccgatagc gcaattaaca atggtgttgc aagctttacc     360

cagcatatca aaaaaagcgg tctgtttctg aaagcaaata gcagcctggc tggtgccggt     420

gaaggtgttg cactgagcca tcaggatcgt cgtaatgatc atgaagttga actggccatt 480

gtgattggta aaaccgcacg taatgttccg cgtgaaaaaa gcctggaata tgttgcaggt 540

tattgcattg gtattgatat gaccgttcgt ggtccggaag aacgtagctt tcgtaaaagt 600

ccggatagct ataccattct gggtccgtgg ctggttaccc gtgatgaaat tgatagtccg 660

ggtgaactgc agatgagcct gaaagttaat ggtgaagttc gtcagaatgc aaataccagc 720

gatctgattc tgggtgtgga agaactggtt gaatttgcaa gcagctttta taccctgcat 780

ccgggtgatg tgattattag cggtacaccg gaaggtgtgg gtccggttaa tcctggtgat 840

gccatgctgg cggaaattga acgtattggc accatgacca ttgcaattcg tagcgttgac 900

tacaaggatg acgatgacaa gtaagatatc gaattcctg 939

<210> 50
<211> 50
<212> DNA
<213> Artificial

<220>
<223> primer

<400> 50
atctgcagct ggtacttaaa gaggtatata atgagcaatc tgcaggatgc 50

<210> 51
<211> 35
<212> DNA
<213> Artificial

<220>
<223> primer

<400> 51
attcccatat ggtacttagc ttgcacgatc gctgc 35

## Description

### Technical Field

The present invention relates to a method for producing a microorganism having a sugar metabolic pathway modified. The present invention also relates to a modified microorganism obtained by the method, and a method for producing an aromatic compound or the like, including culturing the microorganism and recovering an aromatic compound or a derivative thereof from the culture.

### Background Art

For establishment of a sustainable society, there is a demand for change from the society dependent on oil having a high environmental load and feared to be depleted in the future to a biorefinery society dependent on renewable biomass resources, and in recent years, attention is paid to production of aromatic compounds utilizing a microorganism.

In a metabolic pathway of a microorganism, a large number of aromatic compounds can be synthesized through a shikimic acid pathway. A first reaction of the shikimic acid pathway is a condensation reaction, catalyzed by DAHP synthase, from erythrose-4-phosphate (E4P) and phosphoenolpyruvic acid (PEP) to 3-deoxy-D-heptulosonic acid-7-phosphate (DAHP). Subsequently to this reaction, various aromatic compounds including aromatic amino acids are generated via chorismic acid, a common precursor. Chorismic acid can be converted into several aromatic compounds through a small number of steps, and can be a principal compound in a biosynthetic pathway of aromatic compounds.

Salicylic acid corresponding to a chorismic acid derivative is one of the most significant aromatic compounds used in pharmaceuticals, cosmetics, food and the like, and is a precursor used in production of, for example, aspirin and lamivudine (an anti-HIV drug). In recent years, it is reported that salicylic acid can be converted, by a microbial and chemical method, into a raw material of nylon-6,6, adipic acid.

In a living microorganism or the like, salicylic acid can be synthesized through two reactions catalyzed by isochorismate synthase (ICS) converting chorismic acid into isochorismic acid, and by isochorismate pyruvate lyase (IPL) promoting depyruvation of isochorismic acid. In Non Patent Literature 1, a salicylic acid synthetic gene derived from *Pseudomonas aeruginosa* is analyzed, and it is described that salicylic acid can be biosynthesized from chorismic acid through the actions of the above-described two enzymes. Besides, it is reported that salicylic acid was synthesized by expressing, in *E. coli,* EntC, that is, ICS in *E. coli,* and IPL (PchB) derived from *Pseudomonas aeruginosa* (Non Patent Literature 2). The productivity of salicylic acid in these methods is low, however, and a practically effective biosynthesis method has not been developed yet.

### Citation List

### Non Patent Literature

Non Patent Literature 1: Serino L. et al., Mol Gen Genet., 1995, vol. 249, No. 2, pp. 217-228
Non Patent Literature 2: Lin Y. et al., Metab Eng., 2014, vol. 23, pp. 62-69

### Summary of Invention

### Technical Problem

As described above, when a salicylic acid synthase is merely expressed in *E. coli,* satisfactory productivity cannot be obtained. This suggests that high productivity of a target compound cannot be obtained merely by incorporating a desired reaction due to various complicated enzyme reactions in *E. coli.*

### Solution to Problem

The present inventors have examined several strategies for improving a flow of carbon into a biosynthetic pathway of an aromatic compound. In a shikimic acid pathway, utility of PEP and E4P in a cell is one of the most significant factors, and it is important to increase an amount of PEP used in a cell for enhancing an aromatic compound synthetic pathway.

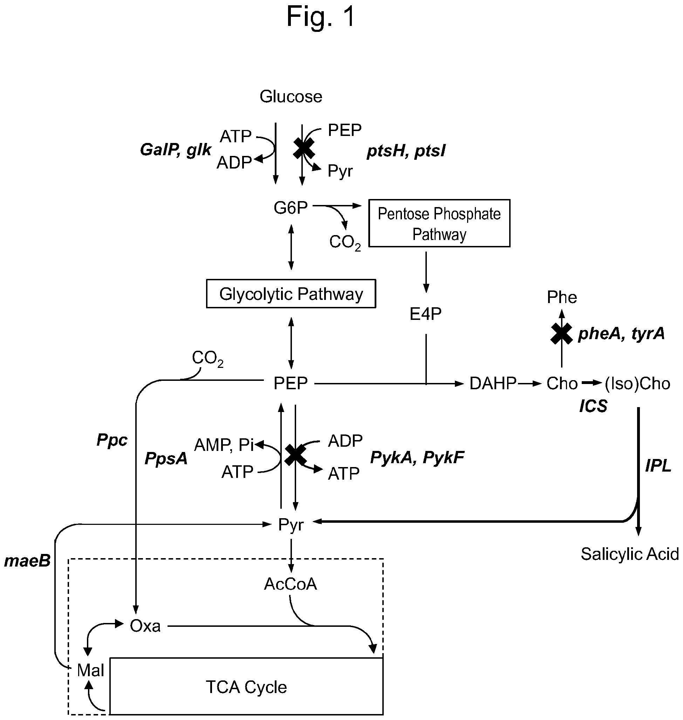

In principal metabolic pathways of a microorganism, there are two reactions consuming PEP. One is incorporation of glucose using a phosphotransferase system (PTS), in which 1 mol of PEP is consumed to be changed into pyruvic acid for incorporating 1 mol of glucose. The other is conversion of PEP into pyruvic acid by pyruvate kinase in a glycolytic pathway (Figure 1).

It is known that phosphoenolpyruvate synthase (ppsA) and transketolase (tktA) are overexpressed to improve the availability of PEP and E4P. Accordingly, introduction of these genes can be a method for increasing the productivity of an aromatic compound, but a reaction catalyzed by tktA is a reversible reaction, and hence the reaction does not proceed merely in a direction accumulating E4P. Besides, even if ppsA is overexpressed, the above-described reaction by PTS and pyruvate kinase proceeds much faster, and a sufficient effect cannot be obtained.

The present inventors thought that it was necessary, for modifying the flow of carbon for the synthesis of an aromatic compound, to modify a metabolic pathway so as not to consume PEP in the above-described two reactions. During various studies, when a gene related to salicylic acid synthesis was introduced and the above-described two reactions converting PEP into pyruvic acid in a cell were inactivated, amounts of salicylic acid and L-phenylalanine generated could be increased by three times and twice, respectively, as compared with those in original strains.

Subsequently, considering that the flow of carbon into the salicylic acid synthesis is competitive with a flow of carbon into L-phenylalanine, the productivity of salicylic acid could be further increased to increase the yield by inactivating a biosynthetic pathway of L-phenylalanine. In this case, a microorganism obtains pyruvic acid mainly through a salicylic acid synthetic pathway.

Through the above-described modification, the productivity of salicylic acid from glucose used as an only carbon source could be improved without introducing the genes (tktA, ppsA and csrB) having been greatly studied for the availability of PEP and E4P.

Furthermore, the resultant strain was used to be applied to production of other chorismic acid derivatives such as 4-hydroxybenzoic acid (PHBA), 3-hydroxybenzoic acid (3HBA), 4-aminobenzoic acid (4ABA), 2-aminobenzoic acid (2ABA), L-tyrosine, phenol, cis,cis-muconic acid (MA), gentisic acid, maleylpyruvic acid and maleic acid, and thus, the versatility thereof could be proved. Accordingly, the cell line produced by the present inventors can be a platform cell line for producing aromatic compounds in a microorganism.

Specifically, the present invention provides the following:
1. A method for producing a microorganism having a sugar metabolic pathway modified, comprising: suppressing the expression of a gene encoding a phosphotransferase system enzyme of the microorganism; suppressing the expression of a gene encoding pyruvate kinase of the microorganism; and introducing, into the microorganism, one or more genes encoding an enzyme that enables the microorganism to synthesize an aromatic compound from chorismic acid or isochorismic acid.
2. The method according to 1 above, further comprising suppressing the expression of a gene encoding an enzyme synthesizing L-phenylalanine from chorismic acid.
3. The method according to 1 or 2 above, wherein the aromatic compound is salicylic acid, and a gene encoding isochorismate synthase and a gene encoding isochorismate pyruvate lyase are introduced.
4. The method according to 1 or 2 above, wherein the aromatic compound is 4-hydroxybenzoic acid, and a gene encoding chorismate pyruvate lyase is introduced.
5. The method according to 1 or 2 above, wherein the aromatic compound is 3-hydroxybenzoic acid, and a gene encoding 3-hydroxybenzoate synthase is introduced.
6. The method according to 1 or 2 above, wherein the aromatic compound is 4-aminobenzoic acid, and a gene encoding aminodeoxychorismate synthase and a gene encoding 4-amino-4-deoxychorismate lyase are introduced.
7. The method according to 1 or 2 above, wherein the aromatic compound is 2-aminobenzoic acid, and a gene encoding anthranilate synthase is introduced.
8. The method according to 1 or 2 above, wherein the aromatic compound is L-tyrosine, and a gene encoding chorismate mutase and a gene encoding prephenate dehydratase are introduced.
9. The method according to 1 or 2 above, wherein the aromatic compound is phenol, and a gene encoding tyrosine phenol lyase is introduced.
10. The method according to 3 above, wherein a gene encoding salicylate 1-monooxygenase and a gene encoding catechol 1,2-dioxygenase are further introduced.
11. The method according to 5 above, wherein one or more genes encoding an enzyme that enables to synthesize gentisic acid, maleylpyruvic acid and/or maleic acid from the 3-hydroxybenzoic acid, are further introduced.
12. A microorganism having a sugar metabolic pathway modified obtained by the method according to any one of 1 to 11 above.
13. The microorganism according to 12 above, wherein the microorganism is selected from bacteria, yeasts and fungi.
14. A method for producing an aromatic compound or a derivative thereof, comprising: culturing the microorganism according to 12 or 13 above; and recovering an aromatic compound or a derivative thereof from the culture.
15. The method according to 14 above, wherein the aromatic compound is salicylic acid, 4-hydroxybenzoic acid, 3-hydroxybenzoic acid, 4-aminobenzoic acid, 2-aminobenzoic acid, L-tyrosine or phenol, and the derivative is muconic acid, gentisic acid, maleylpyruvic acid or maleic acid.
16. The method according to 14 or 15 above, wherein only glucose is added as a carbon source.

The present specification embraces the entire contents of Japanese Patent Application No. 2015-165023 based on which the present application claims the benefit of priority.

### Advantageous Effects of Invention

According to the present invention, a metabolic pathway of a microorganism such as *E*. *coli* is modified to increase the availability of PEP in a cell and to simultaneously inhibit a flow of a carbon source into L-phenylalanine, and thus, a large amount of an aromatic compound can be produced from a carbon source such as glucose. Besides, a cell line found by the present invention can be used to produce various chorismic acid derivatives.

A modified microorganism according to the present invention can produce an aromatic compound, such as PHBA, 3HBA, PABA, 2ABA, tyrosine, phenol, muconic acid, gentisic acid, maleylpyruvic acid or maleic acid, on gram scale under 5 mL test tube culture conditions. According to the present invention, a strain producing muconic acid, a significant dicarboxylic acid, could be successfully obtained. This is the first report on muconic acid production using glucose as a single carbon source.

### Brief Description of Drawings

[Figure 1] Figure 1 illustrates metabolic pathways of salicylic acid generation in *E. coli.*
[Figure 2] Figure 2 schematically illustrates a structure of a plasmid pCFTdeltain.
[Figure 3] Figure 3 illustrates culture characteristics of strains CFT01, CFT11, and CFT31. Changes over time in OD₆₀₀ (Figure 3(A)), glucose concentration (Figure 3(B)), salicylic acid concentration (Figure 3(C)), and L-phenylalanine concentration are illustrated.
[Figure 4] Figure 4 illustrates culture characteristics of a strain CFT51. Changes over time in OD₆₀₀ (Figure 4(A)), glucose concentration (Figure 4(B)), and salicylic acid concentration (Figure 4(C)) are illustrated.
[Figure 5] Figure 5 illustrates the yield of salicylic acid and salicylic acid production ability per unit bacterial cell in the strains CFT01, CFT11, CFT31, and CFT51.
[Figure 6] Figure 6 illustrates culture characteristics of the strains CFT01 and CFT51 in a 2-L jar fermenter, and cell growth (OD₆₀₀) (Figure 6(A)), glucose concentration change (Figure 6(B)) and salicylic acid concentration change (Figure 6(C)) in the strain CFT01, and cell growth (OD₆₀₀) (Figure 6(D)), glucose concentration change (Figure 6(E)) and salicylic acid concentration change (Figure 6(F)) in the strain CFT51 are illustrated.
[Figure 7] Figure 7 illustrates pathways though which various aromatic compounds are generated from chorismic acid.
[Figure 8] Figure 8 illustrates increase in amount of chorismic acid derivatives generated using a strain CFT5. The amounts and the yields of 4-hydroxybenzoic acid (PHBA) (Figure 8(A)), 3-hydroxybenzoic acid (3HBA) (Figure 8(B)), 4-aminobenzoic acid (PABA) (Figure 8(C)), 2-aminobenzoic acid (2ABA) (Figure 8(D)), L-tyrosine (Figure 8(E)), and phenol (Figure 8(F)) generated are respectively illustrated.
[Figure 9] Figure 9 illustrates a pathway for synthesizing maleic acid from chorismic acid via gentisic acid and maleylpyruvic acid.
[Figure 10] Figure 10 illustrates culture characteristics of strains CMtS09 and CMtS10. Figures 10(A) and 10(B) respectively illustrate changes over time in OD₆₀₀ (filled circle) and in glucose concentration (open square) in the strains CMtS09 and CMtS10, and Figures 10(C) and 10(D) respectively illustrate amounts of maleic acid (filled circle), 3-hydroxybenzoic acid (filled square) and gentisic acid (filled triangle) generated in the strains CMtS09 and CMtS10.
[Figure 11] Figure 11 illustrates culture characteristics of strains CMtS091 and CMtS101. Figures 11(A) and 11(B) respectively illustrate changes over time in OD₆₀₀ (filled circle) and in glucose concentration (open square) in the strains CMtS091 and CMtS101, and Figures 11(C) and 11(D) respectively illustrate amounts of maleic acid (filled circle), 3-hydroxybenzoic acid (filled square) and gentisic acid (filled triangle) generated in the strains CMtS091 and CMtS101.

### Description of Embodiments

As described above, the present inventors have designed a novel metabolic pathway for overexpressing an aromatic compound in a microorganism.

Specifically, the present invention provides a method for producing a microorganism having a sugar metabolic pathway modified, including suppressing the expression of a gene encoding a phosphotransferase system enzyme of the microorganism; suppressing the expression of a gene encoding pyruvate kinase of the microorganism; and introducing, into the microorganism, one or more genes encoding an enzyme that allows the microorganism to synthesize an aromatic compound from chorismic acid or isochorismic acid.

The term "sugar metabolic pathway" used herein means various reaction systems *in vivo* for decomposing and converting sugars such as glucose to be used as an energy source and a raw material used in biosynthesis of various compounds, and includes glycolytic pathway, TCA cycle, pentose phosphate pathway, and shikimic acid pathway. For example, biosynthetic pathways of salicylic acid illustrated in Figure 1 are given for explaining modification of a sugar metabolic pathway by the method of the present invention, and it is noted that the "sugar metabolic pathway" used herein is not intended to be limited to these pathways. Those skilled in the art can easily think of a "sugar metabolic pathway" to which the method of the present invention is applicable using a sugar other than glucose, or a degradation product or the like of a sugar.

As illustrated in Figure 1, in the method of the present invention, a microorganism is modified such that chorismic acid (Cho) is first converted into isochorismic acid ((Iso)Cho) by isochorismate synthase (ICS), then pyruvic acid (Pyr) is released from isochorismic acid by isochorismate pyruvate lyase (IPL) to generate salicylic acid in a living microorganism. In the latter reaction, pyruvic acid is released from isochorismic acid simultaneously with the synthesis of salicylic acid. Pyruvic acid is an essential compound for a microorganism, and pyruvic acid obtained during the generation of salicylic acid can be used in the glycolytic pathway. In the method of the present invention, a sugar metabolic pathway originally included in a microorganism is modified, for example, so that pyruvic acid is generated only when salicylic acid is generated, and thus, a microorganism suitable for overexpression of an aromatic compound such as salicylic acid can be obtained.

A gene to be introduced in the method of the present invention can be one that any of microorganisms originally has, but is little or never expressed under usual culture conditions due to various expression control mechanisms and the like. According to the method of the present invention, the amount of a target aromatic compound produced in a microorganism can be dramatically increased.

Almost all microorganisms have a sugar metabolic pathway intended to be modified in the present invention. Accordingly, the microorganism whose sugar metabolic pathway can be modified by the method of the present invention is not especially limited, and can be any microorganism as long as it can be easily cultured and a target compound can be easily recovered therefrom. Examples of the microorganism suitably used include bacteria including bacteria belonging to the genus *Escherichia* such as *Escherichia coli* (*E. coli*), bacteria belonging to the family *Corynebacteriaceae,* bacteria belonging to the genus *Bacillus* such as *Bacillus subtillis,* and actinomycetes belonging to the genus *Streptomyces* and the genus *Streptococcus;* yeasts including yeasts belonging to the genus *Saccharomyces* such as *Saccharomyces cerevisiae* and yeasts belonging to the genus *Pichia* such as *Pichia pastoris*; and fungi belonging to the genus *Aspergillus* such as *Aspergillus oryzae.*

In the case of using *E. coli,* an *E. coli* strain having high phenylalanine productivity is preferred from the viewpoint that chorismic acid can be synthesized with higher productivity because a shikimic acid pathway (a phenylalanine synthetic pathway) is activated therein. Examples of such an *E. coli* strain include strains ATCC31882, ATCC31883 and ATCC31884, among which the strain ATCC31882 is more preferred.

The strain ATCC31882 used as a parent strain in Examples of the present invention is an L-phenylalanine overproducing strain, in which feedback inhibition against L-phenylalanine production has been cancelled. The present inventors focused on the availability of phosphoenolpyruvic acid (PEP) in a cell, and examined to increase the amount of PEP usable for the synthesis of salicylic acid. First, in order to reduce PEP consumption, the present inventors replaced a phosphotransferase system (PTS) by a system of a combination of galactose permease (GalP) and glucokinase (Glk). The obtained modified strain of the present invention had a specific growth rate (µ) of 0.55, the growth rate and glucose consumption rate thereof were substantially equivalent to those of the strain ATCC31882, indicating that a PTS-inactivated strain was successfully produced.

Accordingly, the method of the present invention includes suppressing the expression of a gene encoding a phosphotransferase system enzyme.

The term "gene" used herein means a DNA and an RNA having a polynucleotide sequence encoding a protein, particularly an enzyme, and may be a natural gene, a gene obtained by mutating a natural gene, a chemically synthesized gene, or a gene subjected to any of various modifications well known in the field. As long as an enzyme activity of interest is exhibited, the "gene" includes not only one encoding a full-length protein of an enzyme but also one encoding a functional fragment having the enzyme activity.

The term "phosphotransferase system enzyme" used herein means a group of enzymes in a sugar transport system in microorganisms such as bacteria that transfer a phosphate group of PEP to glucose to generate pyruvic acid and convert glucose into glucose-6-phosphate when transporting a sugar such as extracellular glucose into cells. The generated glucose-6-phosphate is metabolized thereafter in a glycolytic system. The "phosphotransferase system" is sometimes referred to as the "phosphoenolpyruvate: sugar phosphotransferase system" in this field.

The "phosphotransferase system enzyme" includes a plurality of enzymes. In order to prevent the occurrence of an enzyme reaction of the phosphotransferase system enzyme, there is no need to suppress the expression of all the enzymes included in the phosphotransferase system, but expression of a gene encoding one or more of the enzymes, preferably expression of ptsH (for example, NCBI Gene ID: 946886) and ptsI (for example, NCBI Gene ID: 946879) may be suppressed for this purpose.

The term "suppress the expression of a gene" used herein means preventing a protein originally encoded by the gene from being expressed, and includes artificially deleting a part of or all the gene by mutating a genomic gene sequence of the microorganism, and inhibiting transcription and/or translation of the gene, and any methods employed in the field may be employed.

As a method for mutating a gene sequence, gene recombination techniques can be suitably used. A method for deleting a gene is not particularly limited, and for example, a commercially available kit such as Quick & Easy E. coli Gene Deletion Kit (Funakoshi, Tokyo, Japan) can be used as described in Examples (http://www.funakoshi.co.jp/contents/580). PCR may be performed using a gene fragment included in the kit as a template with appropriate primers to disrupt a target gene.

The vector pCFTdeltain used in Examples can be used, for purposes of simultaneously performing disruption of ptsHI and introduction of GalP-Glk, for producing a fragment in which a disrupted fragment and an introduced gene are fused. A part of the sequence is shown in SEQ ID NO:36.

SEQ ID NO: 36
term-P_{Alac0-1}-FRT-gb2-neo-FRT in pCFTdeltain:

GCTAGCCCTAGGTCTAGGGCGGCGGATTTGTCCTACTCAGGAGAGCGTTCACCGACAAACAACAGATAAAACGAAAGGCCCA
GTCTTTCGACTGAGCCTTTCGTTTTATTTGATGCCTCTAGCACGCGGATCCTTTATCATCGTCGTCTTTATAGTCGATATC
AAGCTTGGTCAGTGCGTCCTGCTGATGTGCTCAGTATCTTGTTATCCGCTCACAATGTCAATTGTTATCCGCTCACAATTAA
TTAACCCTCACTAAAGGGCGGCCGCGAAGTTCCTATTCTCTAGAAAGTATAGGAACTTCATTCTACCGGGTAGGGGAGGCGC
TTTTCCCAAGGCAGTCTGGAGCATGCGCTTTAGCAGCCCCGCTGGGCACTTGGCGCTACACAAGTGGCCTCTGGCCTCGCAC
ACATTCCACATCCACCGGTAGGCGCCAACCGGCTCCGTTCTTTGGTGGCCCCTTCGCGCCACCTTCCACTCCTCCCCTAGTC
AGGAAGTTCCCCCCCGCCCCGCAGCTCGCGTCGTGCAGGACGTGACAAATGGAAGTAGCACGTCTCACTAGTCTCGTGCAGA
TGGACAGCACCGCTGAGCAATGGAAGCGGGTAGGCCTTTGGGGCAGCGGCCAATAGCAGCTTTGCTCCTTCGCTTTCTGGGC
TCAGAGGCTGGGAAGGGGTGGGTCCGGGGGCGGGCTCAGGGGCGGGCTCAGGGGCGGGGCGGGCGCCCGAAGGTCCTCCGGA
GGCCCGGCATTCTGCACGCTTCAAAAGCGCACGTCTGCCGCGCTGTTCTCCTCTTCCTCATCTCCGGGCCTTTCGACCTGCA
GCAGCACGTGTTGACAATTAATCATCGGCATAGTATATCGGCATAGTATAATACGACAAGGTGAGGAACTAAACCATGGGAT
CGGCCATTGAACAAGATGGATTGCACGCAGGTTCTCCGGCCGCTTGGGTGGAGAGGCTATTCGGCTATGACTGGGCACAACA
GACGATCGGCTGCTCTGATGCCGCCGTGTTCCGGCTGTCAGCGCAGGGGCGCCCGGTTCTTTTTGTCAAGACCGACCTGTCC
GGTGCCCTGAATGAACTGCAGGACGAGGCAGCGCGGCTATCGTGGCTGGCCACGACGGGCGTTCCTTGCGCAGCTGTGCTCG
ACGTTGTCACTGAAGCGGGAAGGGACTGGCTGCTATTGGGCGAAGTGCCGGGGCAGGATCTCCTGTCATCTCACCTTGCTCC
TGCCGAGAAAGTATCCATCATGGCTGATGCAATGCGGCGGCTGCATACGCTTGATCCGGCTACCTGCCCATTCGACCACCAA
GCGAAACATCGCATCGAGCGAGCACGTACTCGGATGGAAGCCGGTCTTGTCGATCAGGATGATCTGGACGAAGAGCATCAGG
GGCTCGCGCCAGCCGAACTGTTCGCCAGGCTCAAGGCGCGCATGCCCGACGGCGAGGATCTCGTCGTGACCCATGGCGATGC
CTGCTTGCCGAATATCATGGTGGAAAATGGCCGCTTTTCTGGATTCATCGACTGTGGCCGGCTGGGTGTGGCGGACCGCTAT
CAGGACATAGCGTTGGCTACCCGTGATATTGCTGAAGAGCTTGGCGGCGAATGGGCTGACCGCTTCCTCGTGCTTTACGGTA
TCGCCGCTCCCGATTCGCAGCGCATCGCCTTCTATCGCCTTCTTGACGAGTTCTTCTGAGCGGGACTCTGGGGTTCGAATAA
AGACCGACCAAGCGACGTCTGAGAGCTCCCTGGCGAATTCGGTACCAATAAAAGAGCTTTATTTTCATGATCTGTGTGTTGG
TTTTTGTGTGCGGCGCGGAAGTTCCTATTCTCTAGAAAGTATAGGAACTTCCTCGAGCCCTATAGTGAGTCGTATTAGTCGA
C

As a method for inhibiting transcription/translation of a gene, for example, RNA interference by an antisense RNA or siRNA can be used.

Those skilled in the art can appropriately design, synthesize or obtain a vector, an antisense RNA or the like to be used for suppressing the expression of a target gene.

When the expression of a gene encoding a phosphotransferase system enzyme is suppressed in a microorganism to delete the enzyme, a conversion reaction from glucose to glucose-6- phosphate does not occur. Accordingly, for properly generating glucose-6-phosphate, the system is preferably replaced by a system not using PEP and combining galactose permease (GalP) and glucokinase (Glk). Alternatively, another saccharification enzyme or hexokinase can be introduced. An enzyme and an enzyme system to be introduced can be appropriately selected in accordance with a carbon source and the like to be used.

In order to further increase the productivity of salicylic acid, the present inventors inactivated another PEP consuming reaction, that is, conversion from PEP to pyruvic acid in the glycolytic pathway catalyzed by pyruvate kinase (PykF and PykA). In the glycolytic pathway, PEP is converted into pyruvic acid by PykF and PykA with ADP as a cofactor. By deleting the enzyme catalyzing this reaction, pyruvic acid essential for a microorganism can be generated only when salicylic acid is generated.

Accordingly, the method of the present invention includes deleting a gene encoding pyruvate kinase or suppressing the expression thereof. The pykF (for example, NCBI Gene ID: 946179) and the pykA (for example, NCBI Gene ID: 946527) both encode pyruvate kinase catalyzing a reaction for generating pyruvic acid from PEP with ADP as a coenzyme. Since both genes complementarily act in a cell of *E. coli* or the like, the reaction cannot be completely suppressed unless both of these genes are deleted.

The method of the present invention further includes introducing, into the microorganism, one or more genes encoding an enzyme that enables the microorganism to synthesize an aromatic compound from chorismic acid or isochorismic acid.

The "enzyme that enables to synthesize an aromatic compound from chorismic acid or isochorismic acid" differs depending on a target aromatic compound. For example, if salicylic acid is to be synthesized by the microorganism and the method of the present invention, the "enzyme that enables to synthesize an aromatic compound from chorismic acid or isochorismic acid" is isochorismate synthase (ICS) and isochorismate pyruvate lyase (IPL), as described in detail below.

In the production of a compound employing the present invention, an enzyme reaction that a microorganism originally has can be used in some cases. Accordingly, there is no need to introduce genes corresponding to all enzymes necessary for the synthesis of a target compound, but a gene encoding an enzyme catalyzing a part of reactions desired to be promoted may be introduced. Two or more genes can be separately, continuously or simultaneously introduced, if necessary, in accordance with a target compound.

Furthermore, the microorganism and the method of the present invention can be used to synthesize additional compounds from a chorismic acid compound via a specific aromatic compound. Such additional compounds includes a non-aromatic compound, and accordingly, the term "derivative of an aromatic compound" is used herein to also intend such a compound. Alternatively, the term "chorismic acid derivative" is herein used in some cases in the sense that the above-described compound including an aromatic compound can be generated through one or more reactions with chorismic acid used as a starting compound.

An aromatic compound or a derivative thereof that can be synthesized in a microorganism from chorismic acid or isochorismic acid is not especially limited, and examples include salicylic acid, methyl salicylate, acetylsalicylic acid (aspirin), catechol, adipic acid, 1,6-hexanediol, 4-hydroxybenzoic acid (PHBA), 3-hydroxybenzoic acid (3HBA), 2,5-dihydroxybenzoic acid (gentisic acid), maleylpyruvic acid, maleic acid, fumaric acid, 4-aminobenzoic acid (PABA), 2-aminobenzoic acid (2ABA), L-tryptophan, tryptamine, aniline, L-tyrosine, phenol, cis,cis-muconic acid, and trans,trans-muconic acid.

The method of the present invention has been described above in three steps of: (1) suppressing the expression of a gene encoding a phosphotransferase system enzyme of the microorganism; (2) suppressing the expression of a gene encoding a pyruvate kinase of the microorganism; and (3) introducing, into the microorganism, one or more genes encoding an enzyme that enables the microorganism to synthesize an aromatic compound from chorismic acid or isochorismic acid. However, the description is given not for explaining the order of modification of the microorganism, and it should be understood that these steps may be performed in any order or at the same time.

When the intracellular availability of PEP is increased by the method of the present invention, the amounts of salicylic acid and L-phenylalanine generated are both increased as compared with those in an original strain. The present inventors have further found that the productivity of salicylic acid can be further increased to increase the yield by inactivating the biosynthetic pathway of L-phenylalanine.

Accordingly, the method of the present invention further includes deleting a gene encoding an enzyme synthesizing L-phenylalanine from chorismic acid or suppressing the expression of the gene.

Although not especially restrictive, examples of a gene presumed to be introduced for producing an aromatic compound by employing the method of the present invention will now be described.

If the target aromatic compound is salicylic acid, the method of the present invention includes introducing a gene encoding isochorismate synthase and a gene encoding isochorismate pyruvate lyase.

The isochorismate synthase (ICS) is an enzyme converting chorismic acid into isochorismic acid. The gene encoding ICS usable in the present invention is not especially limited, and examples of the genes include menF (NCBI Gene ID: 946712) and entC (NCBI Gene ID: 945511) derived from *E. coli,* pchA (SEQ ID NO:37) derived from *Psudomonas aeroginosa*, AtICS derived from *Arabidopsis thaliana,* and BrICS derived from *Brachypodium distachyon.*

SEQ ID NO: 37
*pchA* from *Pseudomonas aeruginosa*:

ATGAGCCGTCTGGCACCGCTGAGCCAGTGTCTGCATGCACTGCGTGGCACCTTTGAACGTGCAATTGGTCAGGCACAGGCAC
TGGATCGTCCGGTTCTGGTTGCAGCAAGCTTTGAAATTGATCCGCTGGATCCTCTGCAGGTTTTTGGTGCATGGGATGATCG
TCAGACCCCGTGTCTGTATTGGGAACAGCCGGAACTGGCATTTTTTGCATGGGGTTGTGCACTGGAACTGCAGGGTCATGGT
GAACAGCGTTTTGCACGTATTGAAGAAAATTGGCAGCTGCTGTGTGCAGATGCAGTTGTTGAAGGTCCGCTGGCTCCGCGTC
TGTGTGGTGGTTTTCGTTTTGATCCGCGTGGTCCGCGTGAAGAACACTGGCAGGCATTTGCAGATGCCAGCCTGATGCTGGC
AGGTATTACCGTTCTGCGTGAAGGTGAACGTTATCGTGTTCTGTGTCAGCATCTGGCAAAACCGGGTGAAGATGCACTGGCA
CTGGCAGCATATCATTGTAGCGCACTGCTGCGTCTGCGTCAGCCTGCACGTCGTCGTCCGAGTGGTCCGACAGCCGGTGCAC
AGGGTGATGCAAGCGCACAAGAACGTCGTCAGTGGGAAGCAAAAGTTAGTGATGCAGTTAGCAGCGTTCGTCAGGGTCGTTT
TGGTAAAGTTGTTCTGGCACGTACCCAGGCACGTCCGCTGGGTGATATTGAACCGTGGCAGGTTATTGAACATCTGCGTCTG
CAGCATGCCGATGCACAGCTGTTTGCATGTCGTCGTGGTAATGCATGTTTTCTGGGTGCAAGTCCGGAACGTCTGGTTCGTA
TTCGTGCCGGTGAAGCACTGACCCATGCACTGGCAGGCACCATTGCACGTGGTGGTGATGCACAAGAAGATGCACGTCTGGG
TCAGGCACTGCTGGATAGCGCAAAAGATCGTCATGAACATCAGCTGGTTGTTGAAGCAATTCGTACCGCACTGGAACCGTTT
AGCGAAGTTCTGGAAATTCCGGATGCACCGGGTCTGAAACGTCTGGCACGTGTTCAGCATCTGAATACCCCGATTCGTGCCC
GTCTGGCAGATGCCGGTGGTATTCTGCGCCTGCTGCAGGCCCTGCATCCGACACCGGCAGTTGGTGGTTATCCGCGTAGCGC
AGCACTGGATTATATTCGTCAGCATGAAGGTATGGATCGTGGTTGGTATGCAGCACCGCTGGGTTGGCTGGATGGTGAAGGT
AATGGTGATTTTCTGGTTGCACTGCGTAGTGCACTGCTGACACCGGGTCGTGGTTACCTGTTTGCAGGTTGTGGTCTGGTTG
GTGATAGCGAACCGGCACATGAATATCGTGAAACCTGTCTGAAACTGAGCGCAATGCGTGAAGCCCTGAGCGCCATTGGTGG
TCTGGATGAAGTTCCTCTGCAGCGTGGTGTTGCCTAA

The isochorismate pyruvate lyase (IPL) is an enzyme promoting depyruvation of isochorismic acid to convert it into salicylic acid. The gene encoding IPL usable in the present invention is not especially limited, and pchB (SEQ ID NO:38) derived from *Pseudomonas aeroginosa*, MbtI derived from *Mycobacterium tuberculosis*, and Irp9 derived from *Yersinia enterocolitica* are preferred, among which pchB is more preferred.

SEQ ID NO: 38
*pchB* from *Pseudomonas aeruginosa*:

ATGAAAACCCCTGAAGATTGTACCGGTCTGGCAGATATTCGTGAAGCAATTGATCGTATTGATCTGGATATTGTTCAGGCAC
TGGGTCGTCGTATGGATTATGTTAAAGCAGCAAGCCGTTTTAAAGCAAGCGAAGCAGCAATTCCGGCACCGGAACGTGTTGC
AGCAATGCTGCCGGAACGCGCACGTTGGGCTGAAGAAAATGGTCTGGATGCACCGTTTGTTGAAGGTCTGTTTGCACAGATT
ATCCATTGGTATATTGCCGAGCAGATCAAATATTGGCGTCAGACCCGTGGTGCAGCCTAA

If the target aromatic compound is 4-hydroxybenzoic acid, the method of the present invention includes introducing a gene encoding chorismate pyruvate lyase.

The chorismate pyruvate lyase (EC 4.1.3.40) is an enzyme converting chorismic acid into 4-hydroxybenzoic acid and pyruvic acid. The gene usable in the present invention is not especially limited, and an example of the gene includes ubiC (SEQ ID NO:39) derived from *E*. *coli.*

SEQ ID NO: 39
*ubiC* from *Escherichia coli*:

ATGTCACACCCCGCGTTAACGCAACTGCGTGCGCTGCGCTATTGTAAAGAGATCCCTGCCCTGGATCCGCAACTGCTCGACT
GGCTGTTGCTGGAGGATTCCATGACAAAACGTTTTGAACAGCAGGGAAAAACGGTAAGCGTGACGATGATCCGCGAAGGGTT
TGTCGAGCAGAATGAAATCCCCGAAGAACTGCCGCTGCTGCCGAAAGAGTCTCGTTACTGGTTACGTGAAATTTTGTTATGT
GCCGATGGTGAACCGTGGCTTGCCGGTCGTACCGTCGTTCCTGTGTCAACGTTAAGCGGGCCGGAGCTGGCGTTACAAAAAT
TGGGTAAAACGCCGTTAGGACGCTATCTGTTCACATCATCGACATTAACCCGGGACTTTATTGAGATAGGCCGTGATGCCGG
GCTGTGGGGGCGACGTTCCCGCCTGCGATTAAGCGGTAAACCGCTGTTGCTAACAGAACTGTTTTTACCGGCGTCACCGTTG
TACTAA

If the target aromatic compound is 3-hydroxybenzoic acid, the method of the present invention includes introducing a gene encoding 3-hydroxybenzoate synthase.

The 3-hydroxybenzoate synthase (EC 4.1.3.45) is an enzyme converting chorismic acid into 3-hydroxybenzoic acid and pyruvic acid. The gene usable in the present invention is not especially limited, and examples of the genes include hyg5 (SEQ ID NO:40) derived from *Streptomyces hygroscopicus*, and cuv10 (SEQ ID NO:41) derived from *Streptomyces sp.* LZ35.
SEQ ID NO: 40
   *hyg5* from *Streptomyces hygroscopicus*: 
ATGCTGAATCCGAGCAGCCTGGTTCTGAATGGTCTGACCAGCTATTTTGAAAATGGTCGTGCACGTGTTGTTCCGCCTGTTG
GTCGTAATATTCTGGGTGTTGTTAATTATGCCAGCGTTTGTGAATATCCGACCCTGGATCATGGTTATCCGGAACTGGAAAT
TAACATGGTTGCACCGACCGCAGAACCGTTTGCCGAAGTTTGGGTTACCGATGCAGAAAGCGAACATGGTGAACGTGATGGT
ATTACCTATGCACATGATGGCGAATATTTCTTTTGTGCAGGTCGTGTTCCGCCAACCGGTCGTTATACCGAAGCAACCCGTG
CAGCATATGTTACCATGTTTGAACTGCTGGAAGAATTTGGTTATAGCAGCGTTTTTCGCATGTGGAACTTTATTGGTGATAT
CAATCGTGATAATGCCGAAGGCATGGAAGTGTATCGTGATTTTTGTCGTGGTCGTGCCGAAGCATTTGAACAGTGTCGTCTG
GAATTTGATCAGTTTCCGGCAGCAACCGGTATTGGTAGCCGTGGTGGTGGTATTGCATTTTATCTGCTGGCATGTCGTAGCG
GTGGTCATGTTCATATTGAAAATCCGCGTCAGGTTCCGGCATATCATTATCCGAAACGTTATGGTCCGCGTGCACCGCGTTT
TGCACGTGCCACCTATCTGCCGAGCCGTGCAGCCGATGGTGTTGGTGGTCAGGTTTTTGTTAGCGGCACCGCAAGCGTTCTG
GGTCATGAAACCGCACATGAAGGTGATCTGGTTAAACAATGCCGTCTGGCACTGGAAAATATTGAACTGGTTATTAGTGGTG
GTAATCTGGCAGCACATGGTATTAGCGCAGGTCATGGCCTGACCGCACTGCGTAACATTAAAGTTTATGTTCGTCGTAGCGA
AGATGTTCCGGCAGTTCGTGAAATTTGTCGTGAAGCATTTAGTCCGGATGCCGATATTGTTTATCTGACCGTTGATGTTTGC
CGTAGCGATCTGCTGGTTGAAATTGAAGGTGTTGTGATG
SEQ ID NO: 41
   *cuv10* from *Streptomyces sp.* LZ35:

ATGAATCCGAGCAGCCTGGTTCTGAATGGTCTGACCAGCTATTTTGAAAATGGTCGTGCCGGTGGTGTTCCGCCTGCAGGTC
GTAATATTCTGGGTGTTGTTAATTATGCCAGCGTTTGTGAATATCCGACCCTGGATCATGGTTATCCGGAACTGGCCATTAA
TATGGTTGCACCGACCGCAGAACCGTTTGCCGAAGTTTGGGTTACCGATGCCGAAGCAGAACATGGTGAACGTGATGGTATT
ACCTATGCACATGATGGCGAATATTTCTTTTGTGCGGGTCGTGTTCCTCCGACGGGTCGTTATACCGAAGCAACCCGTGCAG
CATATGTTACCATGTTTGAACTGCTGGAAGAATTTGGTTATAGCAGCGTTTTTCGCATGTGGAACTTTATTGGTGATATCAA
TCGTGATAATGCCGAAGGCATGGAAGTGTATCGTGATTTTTGTCGTGGTCGTGCGGAAGCATTTGAACAGTGTCGTCTGGAA
TTTGATCAGTTTCCGGCAGCAACCGGTATTGGTAGCCGTGGTGGTGGTATTGCATTTTATCTGCTGGCATGTCGTAGCGGTG
GTCATGTTCATATTGAAAATCCGCGTCAGGTTCCGGCATATCATTATCCGAAACGTTATGGTCCGCGTGCACCGCGTTTTGC
ACGTGCCACCTATCTGCCGAGCCGTGCAGCCGATGGCGGTGGTGGTCAGGTTTTTGTTAGCGGCACCGCAAGCGTTCTGGGT
CATGAAACCGCACATGAAGGTGATCTGGTTAAACAATGCCGTCTGGCACTGGAAAATATTGAACTGGTTATTAGTGGTGGTA
ATCTGGCAGCACATGGTATTAGCGCAGGTCATGGCCTGACCGCACTGCGTAACATTAAAGTTTATGTTCGTCGTGCAGAAGA
TGTTCCGGCAGTTCGTGAAATTTGTCGTGAAGCATTTAGTCCGGATGCCGATATTGTTTATCTGACCGTTGATGTTTGCCGT
AGCGATCTGCTGGTTGAAATTGAAGGTGTTGTGATGTAA

If the target aromatic compound is 4-aminobenzoic acid, the method of the present invention includes introducing a gene encoding aminodeoxychorismate synthase and a gene encoding 4-amino-4-deoxychorismate lyase.

The aminodeoxychorismate synthase (EC 2.6.1.85) is an enzyme converting chorismic acid and L-glutamine into 4-amino-4-deoxychorismic acid and L-glutamic acid. The 4-amino-4-deoxychorismate lyase (EC 4.1.3.38) is an enzyme converting 4-amino-4-deoxychorismic acid into 4-aminobenzoic acid and pyruvic acid. Examples of the genes encoding these enzymes include, but are not limited to, pabA, pabB and pabC (SEQ ID NO:42) derived from *E. coli.*

SEQ ID NO: 42
*pabC* from *Escherichia coli*:

CTTGATATCGAATTCAAAGAGGAGAAACTGCAGATGTTTCTGATCAACGGTCATAAACAAGAAAGCCTGGCAGTTAGCGATC
GTGCAACCCAGTTTGGTGATGGTTGTTTTACCACCGCACGTGTTATTGATGGTAAAGTTAGCCTGCTGAGCGCACATATTCA
GCGTCTGCAGGATGCATGCCAGCGTCTGATGATTAGCTGTGATTTTTGGCCTCAGCTGGAACAAGAAATGAAAACCCTGGCA
GCAGAACAGCAGAATGGTGTTCTGAAAGTTGTTATTAGCCGTGGTAGCGGTGGTCGTGGTTATAGCACCCTGAATAGCGGTC
CGGCAACCCGTATTCTGAGCGTTACCGCATATCCGGCACATTACGATCGTCTGCGTAATGAAGGTATTACCCTGGCACTGAG
TCCGGTTCGTCTGGGTCGTAATCCGCATCTGGCAGGTATTAAACATCTGAATCGTCTGGAACAGGTTCTGATTCGTAGCCAC
CTGGAACAGACCAATGCAGATGAAGCACTGGTTCTGGATAGCGAAGGTTGGGTTACCGAATGTTGTGCAGCAAACCTGTTTT
GGCGTAAAGGTAATGTTGTTTATACACCGCGTCTGGATCAGGCAGGCGTTAATGGTATTATGCGTCAGTTTTGTATTCGTCT
GCTGGCACAGAGCAGCTATCAGCTGGTTGAAGTTCAGGCAAGCCTGGAAGAAAGTCTGCAGGCAGATGAAATGGTTATTTGT
AATGCACTGATGCCGGTTATGCCGGTGTGTGCATGTGGTGATGTTAGCTTTAGCAGCGCAACCCTGTATGAATATCTGGCAC
CGCTGTGTGAACGTCCGAATGAACAGAAACTGATTAGCGAAGAAGATCTGTAACTGCAGCCCGGGGGA

If the target aromatic compound is 2-aminobenzoic acid, the method of the present invention includes introducing a gene encoding anthranilate synthase.

The anthranilate synthase (EC 4.1.3.27) is an enzyme converting chorismic acid and L-glutamine into anthranilic acid, pyruvic acid and L-glutamic acid. Examples of the genes usable in the present invention include trpE (NCBI Gene ID: 945846) and trpG (NCBI Gene ID: 945109, Microbial Cell Factories 2009, 8:19, Doi: 10.1186/1475-2859-8-19) derived from *E. coli.*

If the aromatic compound is L-tyrosine, the method of the present invention includes introducing a gene encoding chorismate mutase and a gene encoding prephenate dehydratase.

The chorismate mutase (EC 5.4.99.5) is an isomerase converting chorismic acid into prephenic acid. The prephenate dehydratase (EC 4.2.1.51) is an enzyme converting prephenic acid into phenylpyruvic acid, water and carbon dioxide. An example of the gene encoding these enzymes includes tyrA^{fbr} (SEQ ID NO:43) derived from *E. coli.*

SEQ ID NO: 43
*tyrA^{fbr}* from *Escherichia coli*:

ATTAAAGAGGAGAAAGGTACCATGGTTGCTGAATTGACCGCATTACGCGATCAAATTGATGAAGTCGATAAAGCGCTGCTGA
ATTTATTAGCGAAGCGTCTGGAACTGGTTGCTGAAGTGGGCGAGGTGAAAAGCCGCTTTGGACTGCCTATTTATGTTCCGGA
GCGCGAGGCATCTataTTGGCCTCGCGTCGTGCAGAGGCGGAAGCTCTGGGTGTACCGCCAGATCTGATTGAGGATGTTTTG
CGTCGGGTGATGCGTGAATCTTACTCCAGTGAAAACGACAAAGGATTTAAAACACTTTGTCCGTCACTGCGTCCGGTGGTTA
TCGTCGGCGGTGGCGGTCAGATGGGACGCCTGTTCGAGAAGATGCTGACCCTCTCGGGTTATCAGGTGCGGATTCTGGAGCA
ACATGACTGGGATCGAGCGGCTGATATTGTTGCCGATGCCGGAATGGTGATTGTTAGTGTGCCAATCCACGTTACTGAGCAA
GTTATTGGCAAATTACCGCCTTTACCGAAAGATTGTATTCTGGTCGATCTGGCATCAGTGAAAAATGGGCCATTACAGGCCA
TGCTGGTGGCGCATGATGGTCCGGTGCTGGGGCTACACCCGATGTTCGGTCCGGACAGCGGTAGCCTGGCAAAGCAAGTTGT
GGTCTGGTGTGATGGACGTAAACCGGAAGCATACCAATGGTTTCTGGAGCAAATTCAGGTCTGGGGCGCTCGGCTGCATCGT
ATTAGCGCCGTCGAGCACGATCAGAATATGGCGTTTATTCAGGCACTGCGCCACTTTGCTACTTTTGCTTACGGGCTGCACC
TGGCAGAAGAAAATGTTCAGCTTGAGCAACTTCTGGCGCTCTCTTCGCCGATTTACCGCCTTGAGCTGGCGATGGTCGGGCG
ACTGTTTGCTCAGGATCCGCAGCTTTATGCCGACATCATTATGTCGTCAGAGCGTAATCTGGCGTTAATCAAACGTTACTAT
AAGCGTTTCGGCGAGGCGATTGAGTTGCTGGAGCAGGGCGATAAGCAGGCGTTTATTGACAGTTTCCGCAAGGTGGAGCACT
GGTTCGGCGATTACgttCAGCGTTTTCAGAGTGAAAGCCGCGTGTTATTGCGTCAGGCGAATGACAATCGCCAGTAAGGGCC
CCCCCTCGAG

If the target aromatic compound is phenol, the method of the present invention includes introducing a gene encoding tyrosine phenol lyase.

The tyrosine phenol lyase (TPL, EC 4.1.99.2) is an enzyme catalyzing a reaction for converting L-tyrosine and water into phenol, pyruvic acid and ammonia in a stepwise manner. The gene encoding TPL usable in the present invention is not especially limited, and an example of the gene includes tpl (SEQ ID NO:44) derived from *Pasteurella multocida.*

SEQ ID NO: 44
*tp1* from *Pasteurella multocida*:

ATTAAAGAGGAGAAAGGTACCATGAGAAACTATCCTGCAGAACCTTATAAAGTCAAAGCTGTTGAACCGATTGCAATGACGA
CTCGTGAGCAACGTGAAGCCTACATGAAAAAAGCAGGCTACAATACGTTTTTACTCAATTCAGAAGAAGTGTATATTGATTT
ATTAACCGATAGTGGTACCTCAGCCATGAGTGATAAGCAATGGGCGGGTCTTATGATCGGTGATGAAGCTTATGCAGGTAGC
CGTAACTTCATGCATTTACAAGACGTGGTCAGAGAATATTATGGTTTCAAATATGTCGTACCAACTCACCAAGGTCGTGGTG
CAGAAAACTTACTTTCAACCATTATGATCAAACCGGGCGATTATGTACCGGGTAATATGTATTTTACTACAACACGCGCGCA
CCAAGAGCGTAATGGGGCGACTTTCGTTGATATTATTATTGATGAAGCCCATGATTCACAAATTGATTTACCTTTCAAAGGC
AATGTCGATGTGAAGAAATTACAAAAACTGATTGATGAAGTGGGTGCGGATAAAATTCCTTACATCTGTTTAGCGGTTACCG
TAAACTTAGCGGGTGGACAACCTGTGAGTATGGCAAACATGCGTGAAGTAAAAGCATTATGTAGCAAACATGGTATTAAAGT
GATGTTTGATGCCACACGTTGTGTAGAAAATGCGTACTTCATTAAAGAGCGTGAAGCAGAGTATAAAGATGCAACCATCAAA
GATATTCTAAAAGAAATGATGAGTTATGCAGATGGTTGTACCATGAGTGGTAAAAAAGACTGTCTTGTGAATATTGGTGGTT
TCTTATGTATTAATGATGATGATCTTTATCAACAAGCTTGTGAACTCGTGGTGCTCTTTGAAGGGATGCCATCTTATGGTGG
ACTAGCTGGTCGTGATATGGAAGCAATGGCAATTGGTATTACGGAATCTGTCGATTTCCACTATATCCAGCACCGTGTTGCA
CAATGTTACTACCTCGCGGATAAATTAGAAGCGGCGGGTGTGCCGATTGTGAAACCAGTGGGAGGTCATGCGGTATTCTTAG
ATGCGAAGAAATTCCTTCCACATATTCCACAAGAACAATTCCCAGCACAGATGCTTGCTGCGCAAATTTATATTGAGGGTGG
CGTACGTTCAATGGAACGTGGTATTGTATCGGCGGGTCGTGATAAGAAAACCGGTGCAAACCATACGCCTAAATTAGAATTA
GTGCGTTTAACTATCCCACGTCGTGTTTATACTTATGCGCATTTAGATCATGTTGCTGACACCATTATTAAACTCTTTAAAC
ACAGAGATGACATTAAAGGTCTTGATATGGTGTATGAGCCTAAATTATTACGTTTCTTTACGGCGCGTTTTGAACCGAAAGC
GTAAGGGCCCCCCCTCGAG

Besides, when a gene encoding salicylate 1-monooxygenase and a gene encoding catechol 1,2-dioxygenase are further introduced into the microorganism having been modified to be able to synthesize salicylic acid, the microorganism can be caused to synthesize cis,cis-muconic acid.

The salicylate 1-monooxygenase (SMO, EC1.14.13.1) is an enzyme converting salicylic acid into catechol with NADH used as a coenzyme. The catechol 1,2-dioxygenase (CDO, EC 1.13.11.1) is an enzyme converting catechol into cis,cis-muconic acid. The genes encoding these enzymes are not especially limited, and examples of the genes include nahG^{opt} (SEQ ID NO:45) derived from *Pseudomonas putida* TK2440, and catA (SEQ ID NO:46) derived from *Pseudomonas putida* DOT-TIE.
SEQ ID NO: 45
   *nahG^{opt}* from *Pseudomonas putida* TK2440: 
ATTAAAGAGGAGAAAGGTACCATGCAGAACAGTACCAGCGCCCTGAACGTTAGCATCATTGGCGGCGGTATCGCAGGCGTTG
CACTGGCCCTGGACTTATGTCGCCACGCCCACCTGAACGTGCAGCTGTTCGAGGCAGCCCCGGCCTTTGGCGAAGTTGGTGC
CGGTGTTAGCTTCGGCGCCAATGCAGTGCGTGCAATCGCCGGTCTGGGTATCGCAGAGCCGTACGGCAAAATTGCCGACAGT
AATCCGGCCCCGTGGCAGGACATCTGGTTCGAATGGCGCAATGGCCGTGATGCCAAATACCTGGGTTGCAGCGTTGCCGAAG
GCGTTGGTCAAAGCAGTGTGCACCGTGCCGATTTCCTGGACGCTCTGGCTTCTCAGCTGCCGGACGGTATCGCTCAGTTCGG
TAAACGTGCTCAGCGTGTTGAACAGGACGGTGAGCAAGTGCGTGTGACATTCACAGACGGCAGCGAGCACCGCTGCGATCTG
CTGATTGGTGCCGACGGTATCAAGAGTAGCATCCGTGACCACGTGTTACAGGGCCTGAATCAACCGCTGGCAAGCCCGCGTT
TTAGCGGCACCTGCGCCTATCGCGGTCTGATCGATAGCCAGCAGCTGCGTGAGGCCTATCGTGCCCGTGGCGTGGACGAGCA
TCTGATTGACGTGCCGCAGATGTACCTGGGCCTGGACGGCCACATCCTGACCTTCCCGGTTAAACAAGGCCGCCTGATCAAC
GTGGTGGCCTTCATCAGTGACCGCAGCCAACCGAACCCGGTTTGGCCGAGCGATACACCGTGGGTTCGTAATGCCACCCAAG
CCGAGATGCTGGCCGCATTCGAGGGCTGGGATGATGCAGCCCAAGTGCTGCTGGAGTGCATCCCGACCCCTAGTCTGTGGGC
CCTGCACGACCTGGCAGAATTACCGGGCTACGTTCACGGCCGTGTTGGCTTAATCGGCGACGCCGCCCACGCAATGTTACCG
CATCAGGGTGCCGGTGCAGGTCAGGGTCTGGAGGATGCCTGGTTACTGGCCCGCCTGCTGGAAGACCCGAAGGTGCTGGACA
AACGCCCGCAGGCAGTGCTGGATGCCTATGACGCCGTTCGTCGTCCTCGTGCCTGCCGTGTGCAGCGTACCAGCTTCGAGGC
CGGCGAACTGTATGAGTTCCGTGACCCGGCCGTGTTAGCCGACGAGGAGCGTCTGGGCAAAGTTCTGGCCGAACGCTTTGAC
TGGCTGTGGAACCACGACATGCAGGAAGACTTATTACAGGCCCGTGAGCTGCTGGGTTTACGTGCCCAAGCCGCCTAATAGG
GGCCCCCCCTCGAG
SEQ ID NO: 46
   catA from *Pseudomonas putida* DOT-TIE:

GTCGACGGTATCGATCATTAAAGAGGAGAAAGGATGACCGTGAAAATTAGCCATACCGCAGATATTCAGGCCTTTTTAACC
GTGTTGCAGGTCTGGATCATGCAGAAGGTAATCCGCGTTTTAAACAAATTATTCTGCGTGTTCTGCAGGATACCGCACGTCT
GATTGAAGATCTGGAAATTACCGAAGATGAATTTGGCATGCCGTGGATTATCTGAATCGTCTGGGTGGTCGTAATGAAGCA
GGTCTGCTGGCAGCCGGTCTGGGTATTGAACATTTTCTGGATCTGCTGCAGGATGCAAAAGATGCCGAAGCAGGCCTGGGTG
GTGGTACACCGCGTACCATTGAAGGTCCGCTGTATGTTGCGGGTGCACCGCTGGCACAGGGTGAAGCACGTATGGATGATGG
CACCGATCCGGGTGTTGTTATGTTTCTGCAGGGTCAGGTTTTTGATGCAGATGGCAAACCTCTGGCAGGCGCAACCGTTGAT
CTGTGGCATGCAAATACCCAGGGCACCTATAGCTATTTTGATAGCACCCAGAGCGAATTTAATCTGCGTCGTCGTATTATTA
CCGATGCGGAAGGTCGTTATCGTGCACGTAGCATTGTTCCGAGCGGTTATGGTTGTGATCCGCAGGGTCCGACCCAAGAATG
TCTGGACCTGCTGGGTCGTCATGGTCAGCGTCCGGCACATGTTCATTTTTTCATTAGCGCACCGGGTCATCGTCATCTGACC
ACACAGATTAACTTTGCCGGTGATAAATATCTGTGGGATGATTTTGCATATGCCACCCGTGATGGTCTGATTGGTGAACTGC
GTTTTGTTGAAGATGCAGCAGCAGCACGTGATCGTGGTGTTCAGGGTGAACGTTTTGCAGAACTGAGCTTTGATTTTCGCCT
GCAGGGTGCCAAAAGTCCGGATGCAGAAGCCCGTAGCCATCGTCCGCGTGCACTGCAAGAAGGTTAATAGGATATCGAATTC
CTG

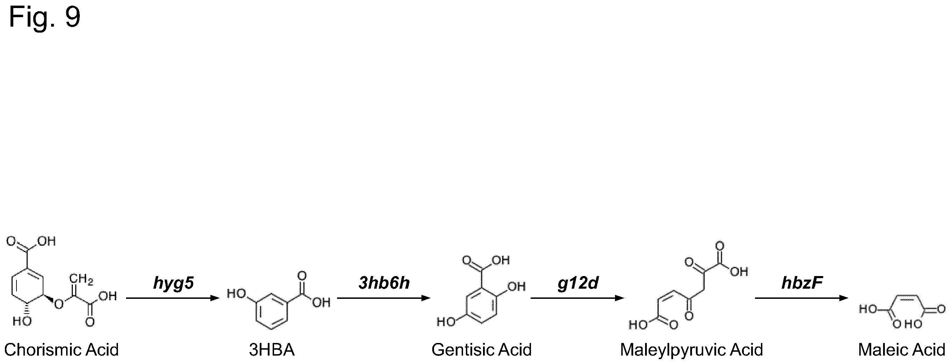

Besides, into the microorganism having been modified to be able to synthesize 3-hydroxybenzoic acid, one or more genes encoding an enzyme that enables to synthesize gentisic acid, maleylpyruvic acid and/or maleic acid from the 3-hydroxybenzoic acid can be introduced. Examples of the genes usable for this purpose include a gene encoding 3-hydroxybenzoate 6-hydroxylase, a gene encoding gentisate-1,2-dioxygenase, and a gene encoding maleylpyruvate hydrolase (Figure 9). The method of the present invention utilizes a biosynthetic pathway that a microorganism originally has, and hence, a gene necessary to be introduced can be varied depending on the type of the microorganism. Alternatively, the above-described genes can be introduced into the microorganism together with a gene encoding the 3-hydroxybenzoate synthase.

The 3-hydroxybenzoate 6-hydroxylase (3H6H, EC 1.14.13.24) is an enzyme converting 3-hydroxybenzoic acid into gentisic acid. The gene encoding this enzyme is not especially limited, and an example of the gene includes 3hb6h (SEQ ID NO:47) derived from *Rhodococcus jostii* RHA1.

SEQ ID NO: 47
*3hb6b* from *Rhodococcus jostii* RHA1:

ATGAGCAATCTGCAGGATGCCCGTATTATCATTGCCGGTGGTGGTATTGGTGGTGCAGCAAATGCACTGGCACTGGCCCAGAAAGG
TGCAAATGTTACCCTGTTTGAACGTGCAAGCGAATTTGGTGAAGTTGGTGCAGGTCTGCAGGTTGGTCCGCATGGTGCACGTATTC
TGGATAGCTGGGGTGTTCTGGATGATGTTCTGAGCCGTGCATTTCTGCCGAAAAATATCGTTTTCGTGATGCCATTACCGCAGAA
GTTCTGACCAAAATTGATCTGGGTAGCGAATTTCGTGGTCGTTATGGTGGTCCGTATTTTGTTACCCATCGTAGCGATCTGCATGC
AACCCTGGTTGATGCAGCACGTGCAGCCGGTGCAGAACTGCATACCGGTGTTACCGTTACCGATGTTATTACCGAAGGTGATAAAG
CAATTGTGAGCACCGATGATGGTCGTACCCATGAAGCAGATATTGCACTGGGTATGGATGGTCTGAAAAGCCGTCTGCGTGAAAAA
ATCAGCGGTGATGAACCGGTTAGCAGCGGTTATGCAGCATATCGTGGCACCACCCCGTATCGTGATGTTGAACTGGATGAAGATAT
TGAAGATGTGGTGGGTTATATTGGTCCGCGTTGTCATTTTATTCAGTATCCGCTGCGTGGTGGTGAAATGCTGAATCAGGTTGCAG
TTTTTGAAAGTCCGGGATTCAAAAACGGCATTGAAAATTGGGGTGGTCCGGAAGAACTGGAACAGGCATATGCACATTGCCATGAA
AATGTTCGTCGCGGTATTGATTATCTGTGGAAAGATCGTTGGTGGCCGATGTATGATCGTGAACCGATTGAAAACTGGGTTGATGG
TCGCATGATTCTGCTGGGTGATGCCGCACATCCGCCTCTGCAGTATCTGGCAAGCGGTGCAGTTATGGCAATCGAAGATGCAAAAT
GTCTGGCAGATTATGCAGCCGAAGATTTTAGCACCGGTGGTAATAGCGCATGGCCTCAGATTCTGAAAGAAGTTAATACCGAACGC
GCACCGCGTTGTAATCGTATTCTGACCACCGGTCGTATGTGGGGTGAACTGTGGCATCTGGATGGCACCGCACGTATTGCACGTAA
TGAACTGTTTCGTACCCGTGATACCAGCAGCTATAAATACACCGATTGGCTGTGGGGTTATAGCAGCGATCGTGCAAGCTAA

The gentisate-1,2-dioxygenase (GDO, EC 1.13.11.4) is an enzyme converting gentisic acid into maleylpyruvic acid. The gene (g12d) encoding this enzyme is not especially limited, and an example of the gene includes mps2 (SEQ ID NO:48) derived from *Rhodococcus sp.* NCIMB 12038.

SEQ ID NO: 48
*mps2* from *Rhodococcus sp.* NCIMB 12038:

ATTAAAGAGGAGAAAGGTACCATGACCGCAACCGAACCGCATGTTGATGATGATCCGGCACTGAAACAGCTGTATACCGATTTTGA
AGCAGAACATCTGAATCCGCTGTGGACACAGCTGGGTGATCTGATGCCGATGACCCCGACCAGCCGTGCAGTTCCGTTTGTTTGGA
AATGGTCAACCCTGTATCCGCTGGCACAGCGTGCCGGTGATCTGGTTCCGGTTGGTCGTGGTGGTGAACGTCGTGCAATTGCCCTG
GCAAATCCGGGTCTGGGTGGTGTTCCGTATGTTACCCCGACCCTGTGGGCAGCAATTCAGTATCTGGGTCCGAAAGAAGTTGCACC
GGAACATCGTCATGCACAGAATGCATTTCGTTTTGTTGTTGAAGGTGAAGGTGTTTGGACCGTTGTTAATGGTGATCCGGTTGCAA
TGCGTCGTGGTGATTTTCTGCTGACACCGGGTTGGCATTTTCATGGTCATCATAATGAAACCGATCAGCCGATGGCATGGATTGAT
GGTCTGGATATCCCGTTTGTTCATTATACCGATACCGGCTTTTTTGAATTTGGCAGCGAAAATGTTACCGATGATAGCACACCGGA
TGTTAGCCGTAGCGAACGTCTGTGGGCACATCCTGGTCTGCGTCCGCTGGTTGGCCTGGATGCAAAAACCAGCAGCCCGATTGCAG
CATATCGTTGGGAACACACCGATGCAGCACTGCGTGAACAGCTGGCACTGGAAGATGAAGGTTATGCAGCAACCACCGAACCGGGT
CATGCAGCAGTTCGTTATACCAATCCGACCACAGGTGGTGATGTTATGCCGACCATTCGTGCAGAATTTCATCGTCTGCGTGCGGG
TGCACATACCCGTCCGCGTCGTGATGTTGGTAGCACCGTTTATCAGGTTTTTGAAGGTAGCGGTCGTTTTGTGCTGGGTGGTCAGA
CCCGTACCGTGGGTAAAGGTGATATGATTGTTGTTCCGAGCTGGACCGAATGGTCAATTGAAGCCGATACCGAATTTGACCTGTTT
GCATTTAGTGATGCACCGATTGTTGAACGTCTGCATTTTCACCGTACCTATATTAGCGAAGGTGCACATCATCATCATCATCATTA
AGGGCCCCCCCTCGAG

The maleylpyruvate hydrolase (MPH, EC 3.7.1.23) is an enzyme converting maleylpyruvic acid into maleic acid. The gene encoding this enzyme is not especially limited, and an example of the gene includes hbzF (SEQ ID NO:49) derived from *Pseudomonas alcaligenes* NCIMB 9867.

SEQ ID NO: 49
*hbzF* from *Pseudomonas alcaligenes* NCIMB 9867:

GTCGACGGTATCGATTTCATTAAAGAGGAGAAAGGTATGAAAATCTGCCGCTTTAATGAAAATCGTCTGGGTGTTGTTGATGGTGA
TGAAGTTCTGGATGTTACCGAAGCACTGAGCGTTCTGCCGAGCTATGAATATCCGCTGCCTGGTTATGATCCGCTGATTAAACATC
TGGATGCACTGCTGGCACGTATTGAAACCCTGATTAAAGATGCACCGCGTATTCTGCTGGCAGATGTTCGTCTGCTGAGTCCGGTT
GCAAATCCGGGTAAAATCATTGCAGCACCGATTAACTATACCCGTCATCTGCAAGAAGTGCTGGCCGATAGCGCAATTAACAATGG
TGTTGCAAGCTTTACCCAGCATATCAAAAAAGCGGTCTGTTTCTGAAAGCAAATAGCAGCCTGGCTGGTGCCGGTGAAGGTGTTG
CACTGAGCCATCAGGATCGTCGTAATGATCATGAAGTTGAACTGGCCATTGTGATTGGTAAAACCGCACGTAATGTTCCGCGTGAA
AAAAGCCTGGAATATGTTGCAGGTTATTGCATTGGTATTGATATGACCGTTCGTGGTCCGGAAGAACGTAGCTTTCGTAAAAGTCC
GGATAGCTATACCATTCTGGGTCCGTGGCTGGTTACCCGTGATGAAATTGATAGTCCGGGTGAACTGCAGATGAGCCTGAAAGTTA
ATGGTGAAGTTCGTCAGAATGCAAATACCAGCGATCTGATTCTGGGTGTGGAAGAACTGGTTGAATTTGCAAGCAGCTTTTATACC
CTGCATCCGGGTGATGTGATTATTAGCGGTACACCGGAAGGTGTGGGTCCGGTTAATCCTGGTGATGCCATGCTGGCGGAAATTGA
ACGTATTGGCACCATGACCATTGCAATTCGTAGCGTTGACTACAAGGATGACGATGACAAGTAAGATATCGAATTCCTG

As a gene intended to be introduced into a microorganism, commercially available products from suppliers such as Invitrogen can be suitably used. Information including the nucleotide sequence of the gene can be obtained by accessing, via internet, database published by National Center for Biotechnology Information (NCBI), USA, or the like, and the gene can be chemically synthesized in some cases.

Each gene can be optionally modified in the nucleotide sequence thereof for retaining or further improving the enzyme activity. Accordingly, as a gene encoding, the isochorismate synthase, for example, a polynucleotide hybridizing, under stringent conditions, to a polynucleotide consisting of a nucleotide sequence as set forth in SEQ ID NO:37, or a polynucleotide consisting of a sequence having a sequence identity of 90% or more, 95% or more, or 99% or more with the nucleotide sequence as set forth in SEQ ID NO:37, can be used.

For introducing a gene, the gene is preferably amplified by polymerase chain reaction (PCR) using primers suitable for the gene. In this case, two or more genes can be simultaneously amplified in a linked form by selecting a template and primers.

In introducing a gene into a microorganism, a gene encoding each of the above-described enzymes can be incorporated into a suitable expression vector. In this case, codon usage is preferably optimized in accordance with the microorganism used as a host.

Those skilled in the art can select an expression vector suitable for gene introduction for using and modifying bacteria, yeasts and fungi in the method of the present invention. An expression vector to be used for gene introduction or modification can be commercially available from suppliers such as Invitrogen, Life Technologies Corporation, and Expressys, or such a commercially available product can be further modified in accordance with purpose.

The term "expression vector" used herein means a self-replicating vector, namely, a vector that is present as an independent substance outside a chromosome, and whose replication does not depend on replication of the chromosome, and for example, the vector can be constructed based on a plasmid. Procedures and a method for constructing such an expression vector can be any of those commonly used in the field of genetic engineering.

In order that a gene is introduced into a microorganism for expressing a target enzyme, the expression vector preferably contains, in addition to a polynucleotide encoding the enzyme, a DNA sequence controlling the expression, a gene marker for selecting transformed *E. coli,* or the like.

DNA sequences controlling the expression include a promoter, an enhancer, a terminator, and a ribosome binding sequence (RBS). Besides, in addition to the DNA sequences controlling the expression, DNA sequences inducing the expression may be contained. An example of such DNA sequences inducing the expression includes lactose operon capable of inducing the expression of a gene disposed downstream when isopropyl-β-D-thiogalactopyranoside (IPTG) is added. A gene marker to be used in the present invention may be appropriately selected in accordance with a method for selecting transformed *E. coli,* and for example, a gene encoding drug resistance, or a gene complementing auxotrophy can be used.

In the case where two or more genes are intended to be introduced, two or more expression vectors respectively containing the genes may be simultaneously or separately introduced, or these genes may be tandemly incorporated into one expression vector. The protein may be expressed in the form of a fusion protein with or without a suitable linker.

In the method of the present invention, the enzyme encoded by the introduced gene can be expressed in a fused form with an additional functional protein or peptide. The additional functional protein or peptide can be fused directly or indirectly to one of or both of the amino terminal or the carboxy terminal of the enzyme. The additional functional protein is not especially limited, and is appropriately selected in accordance with a function required. For example, for purification, detection or the like of a protein, green fluorescent protein (GFP), luciferase protein, Myc-tagged protein, His-tagged protein, hemagglutinin (HA)-tagged protein, FLAG-tagged protein® (Sigma-Aldrich), glutathione-S-transferase (GST) protein and the like can be used.

The present invention also provides a microorganism having a sugar metabolic pathway modified, obtained by the method of the present invention. The microorganism is selected from bacteria, yeasts and fungi.

The present invention further provides a method for producing an aromatic compound or a derivative thereof, including culturing the above-described modified microorganism of the present invention, and recovering an aromatic compound or a derivative thereof from the culture.

Culture conditions for a microorganism are varied depending on the selected microorganism. Those skilled in the art can select appropriate media and culture conditions in accordance with various microorganisms of bacteria, yeasts and fungi, and a medium and culture conditions are not especially limited. For example, culture conditions for *E. coli* are as described below and in Examples.

The medium may be a liquid medium or a solid medium, and is preferably a liquid medium from the viewpoint that a product secreted outside the microorganism can be easily recovered. The medium may contain, in addition to components necessary for culturing the microorganism, a carbon source that can be a raw material of biosynthesis of an aromatic compound or the like intended to be highly produced.

The carbon source is not especially limited, and is preferably glucose, fructose, galactose, mannose, xylose, arabinose, glycerol, glucose-6-phosphate, fructose-6-phosphate, fructose-1,6-bisphosphate, dihydroxyacetone phosphate, glycelaldehyde-3-phosphate, 1,3-bisphosphoglycerate, 3-phosphoglycerate, 2-phosphoglycerate, phosphoenolpyruvic acid, pyruvic acid, acetyl CoA or the like, and is more preferably glucose, that is a major component of biomass, for providing a clean production method using, as a raw material, a renewable resource of biomass. In one embodiment of the present invention, glucose can be added as an only carbon source. The amount of the carbon source to be supplemented to the medium can be appropriately adjusted in accordance with the type of the carbon source, the type of the microorganism, and the like, and is usually 20 to 200 g/L, and preferably 20 to 50 g/L.

Components necessary for culturing the microorganism are not especially limited, and examples include nitrogen sources such as yeast extract, polypeptone, tripeptone, casein and a metabolite thereof, corn steep liquor, soy protein, meat extract, and fish meat extract. Also, a metal salt such as magnesium salt, sodium salt, iron salt, or manganese salt is preferably supplemented. Examples of the metal salt include sodium chloride, sodium dihydrogen phosphate, disodium hydrogen phosphate, potassium dihydrogen phosphate, dipotassium hydrogen phosphate, sodium chloride, iron(II) sulfate, iron(III) sulfate, iron(II) chloride, iron(III) chloride, iron citrate, ammonium iron sulfate, calcium chloride dihydrate, calcium sulfate, magnesium sulfate, zinc sulfate, zinc chloride, copper sulfate, copper chloride, manganese sulfate, and manganese chloride. In addition, as necessary, vitamins and nucleic acid related substances or the like such as biotin, nicotinic acid, thiamine, riboflavin, inositol, and pyridoxine, may be supplemented to the medium.

The medium may be supplemented with a substance for inducing the expression of a protein, such as IPTG, or an antibiotic used for selecting a cell, such as penicillin, β-lactam, macrolide, chloramphenicol, tetracycline, aminoglycoside, ketolide, polyene macrolide, glycopeptide, nucleic acid-based, and pyridone carboxylic acid-based antibiotics.

The culture conditions may be any conditions as long as the microorganism can be grown in the medium and the target compound can be biosynthesized, and those skilled in the art can set the conditions in accordance with the type of the microorganism and the medium to be used, etc. by appropriately adjusting the culture temperature, addition of air, oxygen concentration, carbon dioxide concentration, pH of the medium, shaking speed for the culture, culture time, humidity and the like. Culture temperature is usually 25°C to 40°C, and is preferably 37°C for *E. coli* and the like from the viewpoint that a growth rate can be easily maximized. Suitable pH of the medium for culturing *E. coli* is 7.0, and the shaking speed for the culture is usually 180 to 300 rpm, and preferably 180 to 200 rpm.

A method for recovering an aromatic compound and/or a derivative from the culture medium may be performed by a method usually employed in this field, and is not especially limited. For example, single one of or a combination of methods of filtration, centrifugation and purification using various chromatography, etc. can be employed as necessary.

According to the method of the present invention, various aromatic compounds or derivatives thereof, including salicylic acid, methyl salicylate, acetylsalicylic acid (aspirin), catechol, adipic acid, 1,6-hexanediol, 4-hydroxybenzoic acid (PHBA), 3-hydroxybenzoic acid (3HBA), 2,5-dihydroxybenzoic acid (gentisic acid), maleylpyruvic acid, maleic acid, fumaric acid, 4-aminobenzoic acid (PABA), 2-aminobenzoic acid (2ABA), L-tryptophan, tryptamine, aniline, L-tyrosine, phenol, cis,cis-muconic acid, and trans,trans-muconic acid, can be produced.

### Examples

### <Cell Line and Plasmid>

Cell lines and plasmids used in Examples are shown in Tables 1 and 2 below. For cloning a gene, *E. coli* NovaBlue competent cell (Novagen, Cambridge, MA) was used. It is noted that those shown with no supplier in these tables were produced by the present inventors.

**[Table 1]**

| Cell line | Genotype | Supplier |
|---|---|---|
| Nova Blue | endA1 hsdR17(rK12-mK12+) supE44 thi-I gyrA96 relA1 lac recA1/F' [proAB+ laclq ZΔM15::Tn10(Tetr)]; used for gene cloning. | Novagen |
| ATCC31882 | L-phenylalanine overexpressing strain | ATCC |
| CFT1 | ATCC31882 *ptSHI*::*P*_{A1lacO-1}*₋Glk-GalP* | |
| CFT2 | CFT1Δ*pykF* | |
| CFT3 | CFJ2Δ*pykA* | |
| CFT4 | CFT3Δ*pheA* | |
| CFT5 | CFT4Δ*tyrA* | |
| CFT00 | ATCC31882 harboring pZE12MCS | |
| CFT01 | ATCC31882 harboring pZE12ml | |
| CFT02 | ATCC31882 harboring pZE12el | |
| CFT03 | ATCC31882 harboring pZE12pl | |
| CFT04 | ATCC31882 harboring pTrcBubiC | |
| CFT05 | ATCC31882 harboring pZE12pabABC | |
| CFT06 | ATCC31882 harboring pZA23tyrA^{fbr} | |
| CFT061 | CFT06 harboring pTrcBtpl | |
| CFT07 | ATCC31882 harboring pZE12trpEG | |
| CFT08 | ATCC31882 harboring pTrcBhyg5 | |
| CFT09 | ATCC31882 harboring pZA23ml | |
| CFT091 | CFT09 harboring pZE12nGcA | |
| CFT10 | CFT1 harboring pZE12MCS | |
| CFT11 | CFT1 harboring pZE12ml | |
| CFT30 | CFT3 harboring pZE12MCS | |
| CFT31 | CFT3 harboring pZE12ml | |
| CFT50 | CFT5 harboring pZE12MCS | |
| CFT51 | CFT5 harboring pZE12ml | |
| CFT54 | CFT5 harboring pTrcBubiC | |
| CFT55 | CFT5 harboring pZE12pabABC | |
| CFT56 | CFT5 harboring pZA23tyrA^{fbr} | |
| CFT561 | CFT56 harboring pTrcBtpl | |
| CFT57 | CFT5 harboring pZE12trpEG | |
| CFT58 | CFT5 harboring pTrcBhyg5 | |
| CFT59 | CFT5 harboring pZA23ml | |
| CFT591 | CFT59 harboring pZE12nGcA | |
| CFMt1 | CFT5 harboring pT2t01 | |
| CMtS09 | CFMt1 harboring pS09 | |
| CMtS10 | CFMt1 harboring pS10 | |
| CMtS091 | CMtS09 harboring pA09 | |
| CMtS101 | CMtS10 harboring pA09 | |

**[Table 2]**

| Plasmid | Genotype | Supplier |
|---|---|---|
| pZE12MCS | P_{LlacO1}, colE ori, Amp^{r} | Expressys |
| pTrcHis B | P_{trc}, pBR322 ori, Amp^{r} | Life technologies |
| pZA23MCS | P_{LlacO1}, p15A ori, Km^{r} | Expressys |
| pCFTdeltain | P_{A1lacO-1}, FRT-PGK-gb2-neo-FRT, ColE, Amp^{r} | |
| pCFTdeltain-GG | pCFTdeltain containing *Glk* and *GalP* from *E*. *coli* at the donstream of P_{A1lacO-1} | |
| pZE12l | pZE12MCS containing *pchB* from *P*. *aeruginosa* | |
| pZE12ml | pZE12MCS containing *menF* from *E*. *coli* and *pchB* from *P*. *aeruginosa* | |
| pZE12el | pZE12MCS containing *entC* from *E*. *coli* and *pchB* from *P. aeruginosa* | |
| pZE12pl | pZE12MCS containing *pchA* and *pchB* from *P*. *aeruginosa* | |
| pZE12pabAB | pZE12MCS containing *pabA and pabB* from *E*. *coli* | |
| pZE12pabABC | pZE12MCS containing *pabA, pabB, and pabC* from *E*. *coli* | |
| pZE12trpEG | pZE12MCS containing *trpE, and trpG* from *E*. *coli* | |
| pZE12nahG | pZE12MCS containing *nahG^{opt}* from *P. putida* KT2440. | |
| pZE12nGcA | pZE12nahG containing *catA* from *P.putida* DOT-TIE. | |
| pTrcBubiC | pTrcHis B containing *ubiC* from *E*. *coli* | |
| pTrcBtpl | pTrcHis B containing *tpl* from *P*. *multocida* | |
| pTrcBhyg5 | pTrcHis B containing *hyg5* from *S. hygroscopicus.* | |
| pZA23ml | pZA23MCS containing *menF* from *E*. *coli and pchB* from *P. aeruginosa* | |
| pZA23tyrA^{fbr} | pZA23MCS containing *tyrA^{fbr}* from *E. coli* | |
| pSAK | P_{AlacO1}, SC101 ori, Cm^{r} | |
| pT2t01 | pTrcHis B tandemly containing *mps2, hbzF*, *hyg5,* and *3hb6h* | |
| pS09 | pSAK containing *hyg5* with Ptrc promoter | |
| pS10 | pSAK tandemly containing *hyg5* and *3hb6h* with P_{trc} promoter | |
| pA09 | pZA23MCS containing *hyg5* with Ptrc promoter | |

### <PCR Primer>

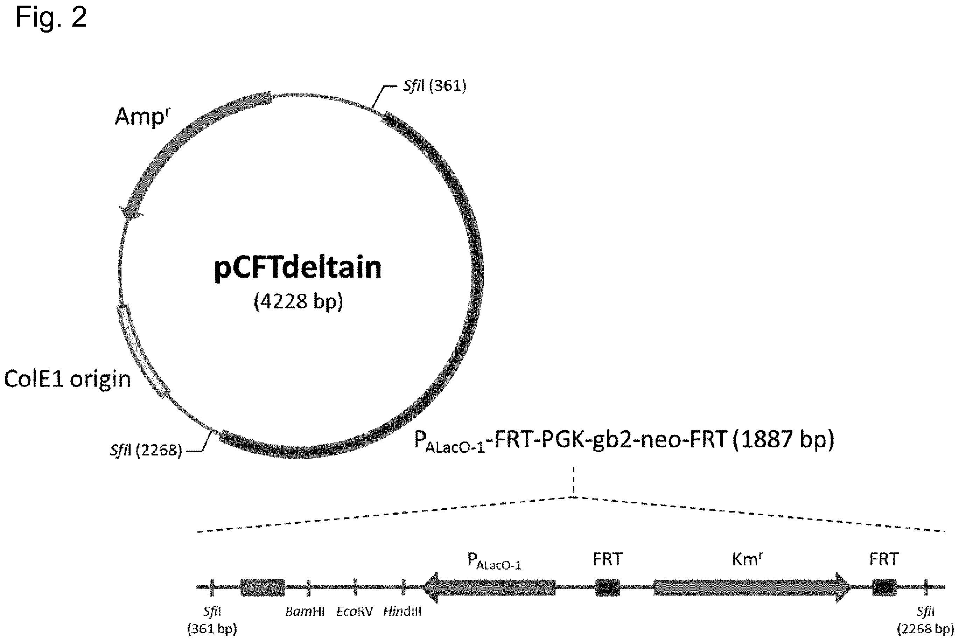

The polymerase chain reaction (PCR) was carried out using KOD FX Neo (TOYOBO, Osaka, Japan) and primer pairs shown in Table 3. Each of the genes was assembled with a corresponding one of the plasmids using Gibson Assembly (New England Biolabs, Ipswich, MA). As the vector pCFTdeltain (Figure 2), a commercially available product was obtained (Invitrogen, San Diego, CA).

**[Table 3]**

| Oligonucleotide primer | Sequence | SEQ ID NO. |
|---|---|---|
| pchB_f | 5'-GTCGACGGTATCGATAAAGAGGAGAAAAAGCTTATGAAAACCCCTGAAGATTG-3' | 1 |
| pchB_r | 5'-CAGGAATTCGATATCTAAATGATGATGATGATGATGGGCTGCACCACGGGTCTGAC-3' | 2 |
| menF_f | 5'-ATTAAAGAGGAGAAAGGTACCATGCAATCACTTACTACGGC-3' | 3 |
| menF_r | 5'-CTCGAGGGGGGGCCCTTACTTGTCATCGTCATCCTTGTAGTCTTCCATTTGTAATAAAGTAC-3' | 4 |
| entC_f | 5'-ATTAAAGAGGAGAAAGGTACCATGGATACGTCACTGGCTGA-3' | 5 |
| entC_r | 5'-CTCGAGGGGGGGCCCTTACTTGTCATCGTCATCCTTGTAGTCATGCAATCCAAAAACGTTCA-3' | 6 |
| pchA_f | 5'-ATTAAAGAGGAGAAAGGTACCATGAGCCGTCTGGCACCGCT-3' | 7 |
| pchA_r | 5'-CTCGAGGGGGGGCCCTTACTTGTCATCGTCATCCTTGTAGTCGGCAACACCACGCTGCAGAG-3' | 8 |
| ubiC_f | 5'-GACGATGACGATAAGATGTCACACCCCGCGTTAAC-3' | 9 |
| ubiC_r | 5'-TGCAGATCTCGAGCTTTAGTACAACGGTGACGCCGGTA-3' | 10 |
| pabA_f | 5'-ATTAAAGAGGAGAAAGGTACCATGATCCTGCTTATAGATAACTACGATTCT-3' | 11 |
| pabA_r | 5'-TTACTTGTCATCGTCATCGTTGTAGTCGGGATGCAGGAAATTAGCCA-3' | 12 |
| pabB_f | 5'-GACGATGACAAGTAAAAAGAGGAGAAACTCGAGATGAAGACGTTATCTCCCGCTGTGATTACT-3' | 13 |
| pabB_r | 5'-CTCGAGGGGGGGCCCTT AAT GATGATGATGATGATGCTTCTCCAGTTGCTTCAGGATACGATTAAC-3' | 14 |
| trpEG_f | 5'-ATTAAAGAGGAGAAAATGCAAACACAAAAACCGAC-3' | 15 |
| trpEG_r | 5'-CTCGAGGGGGGGCCCTTACAGAATCGGTTGCAGCG-3' | 16 |
| tpl_f | 5'-CATCATCATCATGGTATGAGAAACTATCCTGCAGAACCTT-3' | 17 |
| tpl_r | 5'-TCTCGAGCTCGGATCTTACGCTTTCGGTTCAAAACGCGCC-3' | 18 |
| hyg5_f | 5'-CATCATCATCATGGTATGCTGAATCCGAGCAGCCT-3' | 19 |
| hyg5_r | 5'-TCTCGAGCTCGGATCTTACATCACAACACCTTCAA-3' | 20 |
| pTrcB_inv_f | 5' -ACCATGATGATGATGATGAGAACC-3' | 21 |
| pTrcB_inv_r | 5' -GATCCGAGCTCGAGATCTGCAGCT-3' | 22 |
| glk_NI_f | 5'-CAGGACGCACTGACCATGACAAAGTATGCATTAGTCGGTG-3' | 23 |
| glk_NI_r | 5'-TCTTTATCGATACCGTCGACTTAATGATGATGATGATGATGCAGAATGTGACCTAAGGTCT-3' | 24 |
| galP_NI_f | 5'-GTCGACGGTATCGATAAAGAGGAGAAAAAGCTTATGCCTGACGCTAAAAAACA-3' | 25 |
| galP_NI_r | 5'-TGCCTCTAGCACGCGTTACTTGTCATCGTCATCCTTGTAGTCATCGTGAGCGCCTATTTCGC-3' | 26 |
| delta-ptsHI_NI_f | 5'-ATGTTCCAGCAAGAAGTTACCATTACCGCTCCGAACGGTCTGCACACCCGCCTAGGTCTAGGGCGGCGGATTTGT-3' | 27 |
| delta-ptsHI_NI_r | 5'- TTAGCAGATTGTTTTTTCTTCAATGAACTTGTTAACCAGCGTCATTAACTTAATACGACTCACTATAGGGCTCGA-3' | 28 |
| delta-pykF_ f | 5'-ATGAAAAAGACCAAAATTGTTTGCACCATCGGACCGAAAACCGAATCTGAAATTAACCCTCACT AAAGGGCG-3' | 29 |
| delta-pykF_r | 5'-TTACAGGACGTGAACAGATGCGGTGTTAGTAGTGCCGCTCGGTACCAGTGTAATACGACTCACTATAGGGCTC-3' | 30 |
| delta-pykA_f | 5' -A TGTCCAGAAGGCTTCGCAGAACAAAAA TCGTT ACCACGTT AGGCCCAGCAATTAACCCTCACTAAAGGGCG-3' | 31 |
| delta-pykA_r | 5'-TTACTCTACCGTTAAAATACGCGTGGTATTAGTAGAACCCACGGTACTCATAATACGACTCACTATAGGGCTC-3' | 32 |
| delta-pheA_f | 5' -AT G ACATCGGAAAACCCGTTACTGGCGCTGCGAGAGAAAATCAGCGCGCTAATT AACCCTCACTAAAGGGCG-3' | 33 |
| delta-pheA_r | 5'-TCAGGTTGGATCAACAGGCACTACGTTCTCACTTGGGTAACAGCCCAATATAATACGACTCACTATAGGGCTC-3' | 34 |
| delta-tyrA_r | 5'-AGCCGCTTTGGACTGCCTATTTATGTTCCGGAGCGCGAGGCATCTATGTTTAATACGACTCACTATAGGGCTC-3' | 35 |
| 3hb6h_f | 5'-ATCTGCAGCTGGTACTTAAAGAGGTATATAATGAGCAATCTGCAGGATGC-3' | 50 |
| 3hb6h_r | 5'-ATTCCCATATGGTACTTAGCTTGCACGATCGCTGC-3' | 51 |

### <Culture Conditions>

For production of salicylic acid in a 5 mL test tube, an M9 minimal medium was used. The M9 minimal medium contains, per liter, 20 g of glucose, 0.5 g of NaCl, 17.1 g of Na₂HPO₄·12H₂O, 3 g of KH₂PO₄, 2 g of NH₄Cl, 246 mg of MgSO₄·7H₂O, 14.7 mg of CaCl₂·2H₂O, 2.78 mg of FeSO₄·7H₂O, 10 mg of thiamine hydrochloride, 40 mg of L-tyrosine and 40 mg of L-tryptophan (ATCC31882 exhibits L-tyrosine and L-tryptophan auxotrophy). As necessary, ampicillin, kanamycin and/or chloramphenicol were added to respective final concentrations of 100, 50, and 15 µg/mL. In culturing a strain CFT5, L-phenylalanine was added to a final concentration of 100 mg/L. Besides, in pre-culturing strains CFT3 and CFT5, 0.2% sodium pyruvate was supplemented to the medium for promoting the growth. A pre-culture medium was inoculated in the M9 medium put in the 5 mL test tube at an initial optical density (OD₆₀₀) of 0.05. The test tube culture was carried out at 37°C with shaking at 180 rpm. The medium was first supplemented with 0.1 mM IPTG.

Production of PHBA, PABA, 2ABA, L-tyrosine, 3HBA, phenol, MA and maleic acid was carried out by 5 mL test tube culture. The production of PHBA and L-tyrosine was performed in an M9 medium supplemented with 0.1% sodium pyruvate, the production of 2ABA and maleic acid was performed in an M9Y medium supplemented with 0.5% yeast extract, and the production of PABA, 3HBA, phenol and muconic acid was performed in an M9Y medium supplemented with 0.1% sodium pyruvate and 0.5% yeast extract. Each of the pre-culture medium was inoculated in the medium put in a 5 mL test tube at an initial optical density (OD₆₀₀) of 0.05. The test tube culture was carried out at 37°C with shaking at 180 rpm.

Batch culture was carried out by a method usually employed in this field in consideration of the above-described culture conditions. The batch culture of salicylic acid will be more specifically described in Example 5 below.

### <Analysis of Various Products>

Glucose concentration in a culture supernatant was measured using Glucose CII test Wako (Wako, Kyoto, Japan). In the case where sodium pyruvate was added, not only an amount of glucose consumed but also an amount of sodium pyruvate consumed was taken into consideration for evaluating the yield ([amount of compound generated] - mol/[amounts of glucose and pyruvic acid consumed] - mol%).

GC-MS was carried out using GCMS-QP2010 Ultra (Shimadzu, Kyoto, Japan) equipped with a CP-Sil 8 CB-MS capillary column (30 m x 0.25 mm x 0.25 µm; Agilent). Helium was used as a carrier gas, and a flow rate of 2.1 ml/min was retained. An injection amount was set to 1 µl (split ratio of 1:10).

The amounts of salicylic acid and muconic acid generated were quantitatively determined as follows. An initial temperature of an oven was set to and retained for 1 minute at 150°C, and the temperature was increased up to 240°C at 12°C/min, further increased up to 300°C at 120°C/min, and retained at 300°C for 3 minutes. The total measurement time was set to 10 minutes. Other setting conditions were: an interface temperature of 250°C, an ion source temperature of 200°C, and an electron ionization voltage (EI) of 70 eV.

Dried salicylic acid and muconic acid were derivatized for analysis, in 50 µL of N-methyl-N-trimethylsilyltrifluoroacetamide (MSTFA) and 20 µL of pyridine at 80°C for 60 minutes. Pimelic acid was used as an internal standard.

The amounts of PHBA, L-phenylalanine, and L-tyrosine generated were quantitatively determined as follows. An initial temperature of an oven was set to and retained for 5 minutes at 150°C, and the temperature was increased up to 300°C at 10°C/min, and retained at 300°C for 5 minutes. The total measurement time was set to 25 minutes. Other setting conditions were: an interface temperature of 250°C, an ion source temperature of 200°C, and an electron ionization voltage (EI) of 70 eV.

4-Hydroxybenzoic acid, 4-aminobenzoic acid, L-phenylalanine, and L-tyrosine were derivatized for analysis, in 30 µL of N-(tert-butyldimethylsilyl)-N-methyl-trifluoroacetamide (MTBSTFA) and 30 µL of N,N-dimethylformamide at 80°C for 60 minutes. Cycloleucine was used as an internal standard.

Concentrations of 2ABA, PABA, 3HBA and phenol were measured by high-performance liquid chromatography (HPLC; Shimadzu, Kyoto, Japan) using a 5C₁₈-AR-II column (Nacalai Tesque, Kyoto, Japan). Measurement conditions of 30°C and a flow rate of 1.2 ml/min were employed. As a solvent system, phosphoric acid aqueous solution (50 mM, pH 2.5) was used as Solvent A, and acetonitrile was used as Solvent B. Concentration gradient was started from 70% Solvent A and 30% Solvent B, a 50:50 mixture of Solvents A and B was used from 3 min to 7 min, and a 70:30 mixture of Solvents A and B was subsequently used from 7 min to 10 min.

The concentrations of 2ABA, PABA and 3HBA were measured using a 254 nm UV absorbance detector (SPD-20AV, Shimadzu, Kyoto, Japan). For phenol, absorbance at 270 nm was detected. A culture supernatant was obtained by centrifuging a culture fluid at 21,880 x g for 20 minutes, and the resultant was used in the HPLC. As an internal standard for the measurement of 2ABA, PABA and 3HBA, benzoic acid was used, and for phenol, 2ABA was used.

Concentration of maleic acid was measured in the same manner as described above using a culture supernatant separated by centrifuging a culture fluid at 21,880 x g for 20 minutes. The culture supernatant was analyzed by the high-performance liquid chromatography (HPLC; Shimadzu, Kyoto, Japan) using a 5C₁₈-PAQ column (Nacalai Tesque, Kyoto, Japan). A column was operated at 30°C, and a flow rate was set to 1.2 ml/min. As a solvent system, phosphoric acid aqueous solution (50 mM, pH 2.5) was used as Solvent A, and acetonitrile was used as Solvent B. Concentration gradient was started from 100% Solvent A (0 min to 3 min), Solvent A was gradually changed to a 50:50 mixture of Solvents A and B (3 min to 6 min), and the 50:50 mixture of Solvents A and B was maintained (6 min to 7 min), and was subsequently gradually changed to 100% Solvent A (7 min to 13 min). The concentration of a product was measured by detecting 210 nm UV absorbance (SPD-20AV, Shimadzu, Kyoto, Japan). Fumaric acid was used as an internal standard.

### [Example 1] Production of Gene-deletion Cell Line

For producing a chorismic acid derivative, attempts were made to transform *E. coli.* As a parent strain, L-phenylalanine overproducing strain (ATCC31882) of *E. coli* was used.

Gene fragments encoding GalP and Glk were amplified by the PCR using a genomic DNA of *E. coli* MG1655 (ATCC700926) as a template, and using glk_NI_f (SEQ ID NO:23) and glk_NI_r (SEQ ID NO:24), and galP_NI_f (SEQ ID NO:25) and galP_NI_r (SEQ ID NO:26) as respective primers. The amplified fragments were tandemly inserted into the HindIII and BamHI sites of the pCFTdeltain (Figure 2). The obtained plasmid was designated as pCFTdeltain-GG.

A gene deletion operation was carried out using Quick & Easy E. coli Gene Deletion Kit (Funakoshi, Tokyo, Japan) according to a protocol provided in instructions thereof.

A fragment for disrupting ptsHI and introducing P_{a1lacO-1}-Glk-Galp was obtained through amplification by PCR using pCFTdeltain-GG as a template, and using delta-ptsHI_NI_f (SEQ ID NO:27) and delta-ptsHI_NI_r (SEQ ID NO:28) as primers. Fragments for inactivating pykF, pykA and pheA genes were similarly obtained through amplification by PCR using the genomic DNA of *E. coli* MG1655 as a template, and primers shown in Table 3 (SEQ ID NOS:29 to 34).

A gene fragment for deletion and a recombinase were introduced into a cell by electroporation using Gene Pulser II (Bio-Rad Laboratories). Gene inactivated cell lines obtained based on the strain ATCC31882 are herein referred to as strains CFT1 to CFT5.

As shown in Table 1, the strain CFT1 has genes encoding Glk and GalP, and contains ptsHI deletion. Accordingly, in the strain CFT1, originally contained PTS is replaced by a system of a combination of galactose permease (GalP) and glucokinase (Glk).

The strain CFT2 lacks pykF in addition to the mutation in the strain CFT1. The strain CFT3 lacks pykA in addition to the mutation in the strain CFT2. Both pykF and pykA encode pyruvate kinase catalyzing a reaction for generating pyruvic acid from PEP with ADP as a coenzyme, and since both genes complementarily act in a cell of *E. coli* or the like, the reaction cannot be completely suppressed unless both of these genes are deleted. In the strain CFT3, two reaction pathways producing pyruvic acid are blocked.

The strain CFT4 lacks pheA in addition to the mutation in the strain CFT3. Accordingly, the strain CFT4 is modified such that not only the reaction producing pyruvic acid is deleted but also a phenylalanine synthesis reaction cannot be performed. The strain CFT5 lacks tyrA in addition to the mutation in the strain CFT4, and is modified such that a tyrosine synthesis reaction cannot be performed. The synthesis of phenylalanine can be completely suppressed, only when both genes are disrupted.

### [Example 2] Production of Salicylic Acid Synthesizing Cell Line

The strain ATCC31882 was used for introducing genes encoding ICS and IPL to increase salicylic acid synthetic ability thereof. In this example, menF, entC and pchA were used as genes encoding ICS, and pchB was used as a gene encoding IPL.

As pchA and pchB gene fragments derived from *P. aeruginosa*, commercially available products (SEQ ID NOS:37 and 38, Invitrogen) were obtained, and codon usage of pchA and pchB was optimized for *E. coli.*

A gene fragment encoding pchB was amplified by PCR using a synthetic gene fragment resulting from codon optimization as a template, and using pchB_f (SEQ ID NO:1) and pchB_r (SEQ ID NO:2) as primers. The amplified fragment was inserted into the HindIII site of pZE12MCS (Expressys). The obtained plasmid was designated as pZE12I.

Gene fragments encoding menF and entC were respectively amplified by PCR using the genomic DNA of *E. coli* MG1655 as a template, and using menF_f (SEQ ID NO:3) and menF_r (SEQ ID NO:4), and entC_f (SEQ ID NO:5) and entC_r (SEQ ID NO:6) as respective primers. Each of the amplified fragments was inserted into the KpnI site of the pZE12I containing the gene encoding pchB. The thus obtained plasmids were designated as pZE12mI and pZE12eI.

A gene fragment encoding pchA was amplified by PCR using the synthetic gene fragment resulting from codon optimization as a template, and using pchA_f (SEQ ID NO:7) and pchA_r (SEQ ID NO:8) as primers. The amplified fragment was inserted into the KpnI site of pZE12I containing the gene encoding pchB. The obtained plasmid was designated as pZE12pI.

A gene fragment encoding menF-pchB (fusion protein of menF and pchB) was amplified by PCR using pZE12mI as a template, and using menF_f (SEQ ID NO:3) and pchB_r (SEQ ID NO:2) as primers. The amplified fragment was inserted into the KpnI site of pZA23MCS (Expressys). The obtained plasmid was designated as pZA23mI.

Transformation of strains using each plasmid was carried out using Gene Pulser II (Bio-Rad, Hercules, CA). As necessary, 100 µg/L ampicillin and 50 µg/L kanamycin were added to the medium.

Transformants obtained by introducing the above-described plasmids pZE12mI, pZE12eI, pZE12pI and pZA23mI into the strain ATCC31882 were herein designated as strains CFT01, CFT02, CFT03, and CFT09, respectively. Each of these transformants was cultured in an M9 minimal medium containing glucose as a carbon source. IPTG was added to an initial medium (OD₆₀₀ = 0.05).

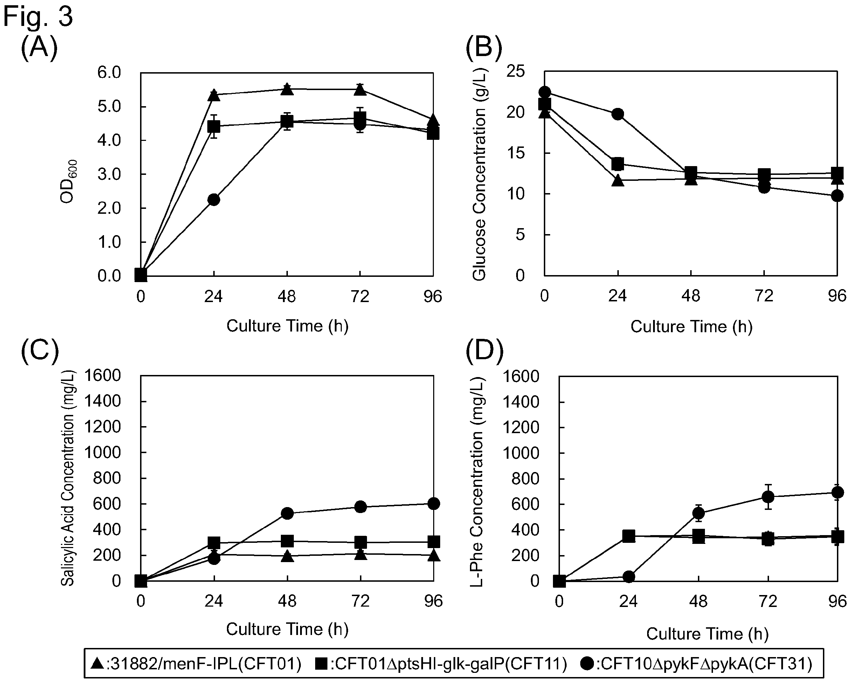

The culture characteristics of the strain CFT01 are shown in Figure 3. The amounts of salicylic acid and L-phenylalanine generated by the strain CFT01 having menF and pchB were as large as 210 and 350 mg/L, respectively, and thus, salicylic acid could be produced using glucose as an only carbon source. These amount values were larger than those obtained in the strains CFT02, CFT03 and CFT09. Accordingly, it was determined that menF was to be used as the gene encoding ICS in the subsequent experiments.

### [Example 3] Introduction of Salicylic Acid Biosynthetic Pathway

Each plasmid produced in Example 2 was introduced into the mutant strains produced in Example 1 instead of the strain ATCC31882 for examining salicylic acid generation ability.

pZE12mI was introduced into the strain CFT1 having genes encoding Glk and GalP and containing ptsHI deletion so as to produce a strain capable of expressing menF and pchB. The obtained strain is herein designated as a strain CFT11.

The strain CFT11 was cultured for evaluation in an M9 minimal medium containing glucose as an only carbon source, in the same manner as the strain CFT01. The culture characteristics of the strain CFT11 are illustrated in Figure 3. The amounts of salicylic acid and L-phenylalanine generated were as large as 311 and 358 mg/L, respectively. Although the productivity of salicylic acid was increased by 1.5 times as compared with that in the strain CFT01, the productivity of L-phenylalanine was substantially the same.

Next, the pZE12mI was introduced into the strain CFT3 containing both pykF and pykA deletion in addition to the mutation in the strain CFT1 so as to produce a strain capable of expressing menF and pchB. The obtained strain is herein designated as a strain CFT31.

The strain CFT31 was cultured in an M9 minimal medium containing glucose as an only carbon source, and as a result, the amounts of salicylic acid and L-phenylalanine generated were as large as 603 and 694 mg/L, respectively, as illustrated in Figure 3. As shown in Figures 3(A) and 3(B), the cell growth and the glucose consumption rate of the strain CFT31 tended to be slightly lower than those of the strains CFT01 and CFT11, but the amount of salicylic acid generated in the strain CFT31 was larger than those in the other strains.

### [Example 4] Inactivation of L-phenylalanine Production Ability

The pZE12mI was introduced into the strain CFT5 containing pheA and tyrA deletion in addition to the mutation in the strain CFT3 so as to produce a strain capable of expressing menF and pchB. The obtained strain is herein designated as a strain CFT51.

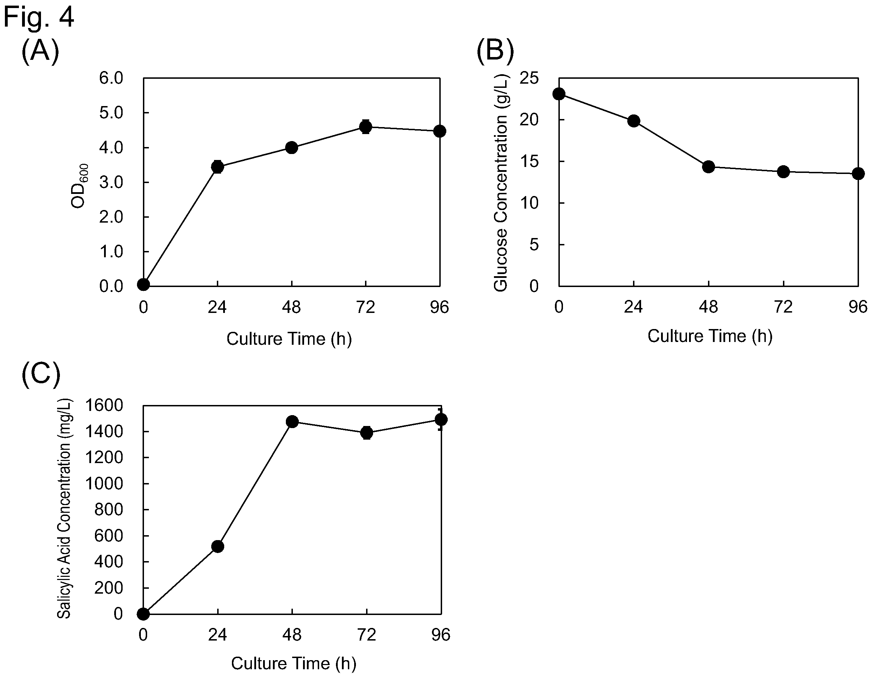

The strain CFT51 was cultured in an M9 minimal medium containing glucose as an only carbon source. Figure 4 illustrates the culture characteristics of the strain CFT51. As a result, the amount of salicylic acid generated was as large as 1,480 mg/L (about 1.5 g/L) after the culture for 48 hours. This amount value was increased by about 7.5 times as compared with that of the strain CFT01, which is capable of expressing menF and pchB but not subjected to the operation for blocking other pathways. On the other hand, generation of L-phenylalanine was not observed (data is now shown).

In order to examine the amount of byproducts generated except for salicylic acid and L-phenylalanine in each of the produced cell lines, amounts of major byproducts of *E. coli* culture, organic acids in a culture supernatant, were measured (Table 4).

**[Table 4]**

| Strain | Time (h) | Amount of organic acids generated (mM) | | | | |
|---|---|---|---|---|---|---|
| | | Pyruvic acid | Lactic acid | Formic acid | Acetic acid | Malic acid |
| CFT01 | 24 | 9.26 ± 0.45 | 4.93 ± 0.87 | 0.19 ± 0.04 | 18.49 ± 0.5 | ND |
| | 96 | 9.22 ±0.74 | 6.06 ± 0.97 | 0.23 ± 0.02 | 19.51 ± 0.48 | ND |
| | | | | | | |
| CFT11 | 24 | 5.93 ± 1.32 | 1.12 ± 0.08 | 0 | 23.27 ± 3.8 | ND |
| | 96 | 4.58 ± 0.92 | 0.37 ± 0.00 | 0.06 ± 0.1 | 30.6 ± 3.31 | ND |
| | | | | | | |
| CFT31 | 24 | ND | ND | ND | ND | ND |
| | 96 | ND | ND | 1.02 ± 0.15 | 6.7 ± 0.24 | 1.06 ± 0.06 |
| | | | | | | |
| CFT51 | 24 | ND | ND | ND | ND | ND |
| | 96 | ND | 0.28 ±0.19 | 0.30 ± 0.05 | 7.4 ± 0.51 | 0.32 ± 0.04 |

As shown in Table 4, acetic acid and pyruvic acid were principally generated in the strains CFT01 and CFT11, and the amounts of generated acetic acid were as large as 19.5 and 30.6 mM (1.17 and 1.84 g/L), respectively. On the other hand, although a small amount of acetic acid was generated in the strains CFT31 and CFT51, no pyruvic acid was accumulated in the culture supernatants, and the generation of pyruvic acid was reduced due to disruption of the reaction converting PEP into pyruvic acid. A total amount of organic acids generated in each of the strains CFT31 and CFT51 was less than 8.5 mM, and was dramatically reduced as compared with that in the strains CFT01 and CFT11.

Based on the above results, it is presumed, that the production of the byproduct organic acids is suppressed in the strains CFT31 and CFT51, and glucose is efficiently converted into salicylic acid. This is probably because pyruvic acid is mildly supplied due to the inactivation of pykF and pykA, and because the production of organic acids such as lactic acid and acetic acid derived from pyruvic acid is reduced.

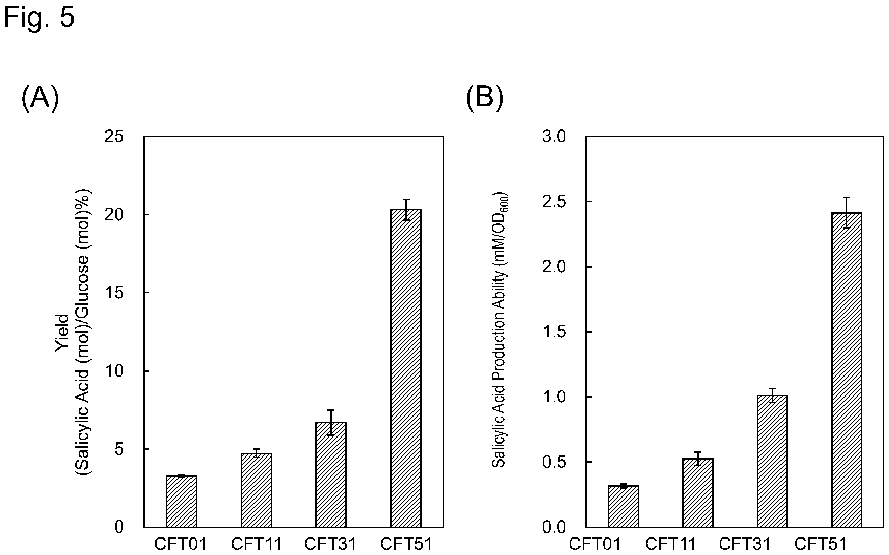

Figure 5 illustrates yields in the generation of salicylic acid from glucose and salicylic acid production ability per OD₆₀₀ in the strains CFT01, CFT11, CFT31 and CFT51. The yield of salicylic acid and the production ability per OD₆₀₀ in the strain CFT51 are increased by 6.2 times and 7.6 times, respectively, as compared with those in the strain CFT01, and thus, it was proved that the productivity of salicylic acid was dramatically increased.

### [Example 5] Production of Salicylic Acid by Batch Culture

The obtained cell lines were used for producing salicylic acid by mass culture.

Batch culture was carried out in a 2.0 L jar fermenter (working volume of 400 mL). For production of salicylic acid in the jar fermenter, an M9Y medium consisting of an M9 medium containing 0.4% yeast extract was used. 4 mL of a pre-culture medium was put in the jar fermenter having been charged with 400 mL of the medium. In order to maintain the pH at 7.0 during the culture, NH₄OH was automatically added. Dissolved oxygen (DO) concentration was retained at 3.0 ppm or higher by automatically controlling the stirring speed and supplying oxygen at 37°C. The initial glucose concentration was 40 g/L. After the culture for 5 hours, 0.1 mM IPTG was supplemented to the medium.

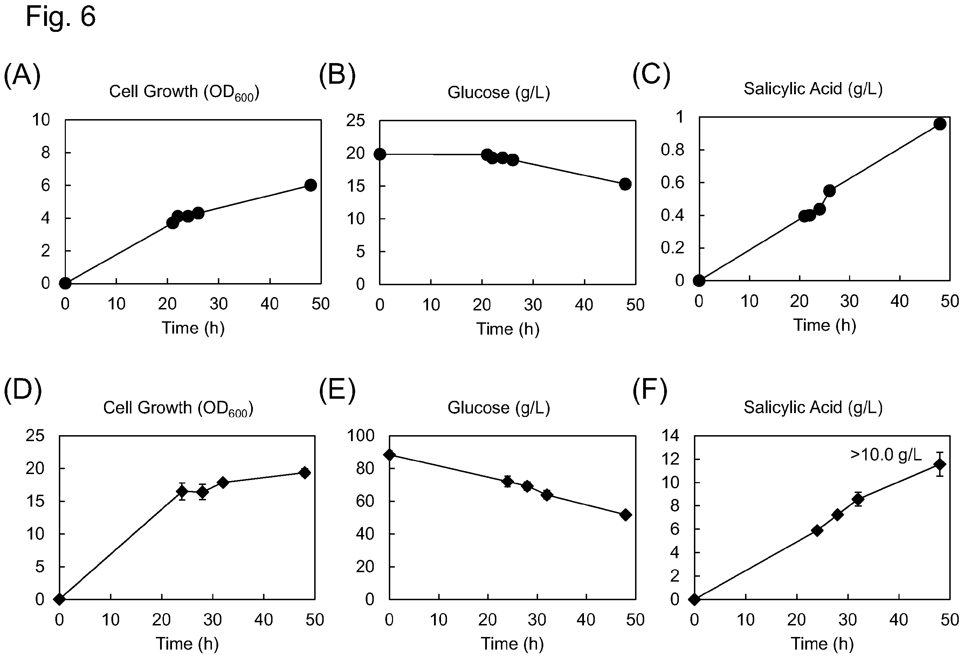

Figure 6 illustrates culture characteristics of the strains CFT01 and CFT51. The cell growth and the glucose consumption rate in the strain CFT51 were improved as compared with those in the strain CFT01 (Figures 6(A), 6(B), 6(D) and 6(E)). After the culture for 48 hours, the amount of salicylic acid generated in the strain CFT01 was as small as 0.9 g/L, but the amount of salicylic acid generated in the strain CFT51 was as large as about 11 g/L (Figures 6(C) and 6(F)).

The productivity beyond 10 g/L in the strain CFT51 is at the world's highest level (14 times as high as that of the original strain) in an aromatic compound production test performed by batch culture using *E. coli.* The production of salicylic acid using glucose as an only carbon source has not been reported, and the production amount was higher than the past reported production amount using glucose and glycerol as carbon sources.

### [Example 6] Generation Promotion of 4-Hydroxybenzoic Acid

It was presumed that chorismic acid is accumulated in a cell in the strain CFT5 corresponding to the parent strain of the strain CFT51. Accordingly, the present inventors next attempted to produce another chorismic acid derivative based on the strain CFT5, and could verify the versatility of this strain, and construct a platform for producing aromatic compounds.

First, generation of a structural isomer of salicylic acid, 4-hydroxybenzoic acid (PHBA), was examined. In order to enable PHBA to be generated from chorismic acid, a gene (SEQ ID NO:39, ubiC) encoding chorismate pyruvate lyase (EC 4.1.3.40) derived from *E. coli* was introduced.

A gene fragment encoding ubiC was amplified by the PCR using the genomic DNA of *E. coli* MG1655 as a template, and using ubiC_f (SEQ ID NO:9) and ubiC_r (SEQ ID NO:10) as primers. The amplified fragment was inserted into the BamHI site of pTrcHis B (Life Technologies Corporation). The obtained plasmid was designated as pTrcBubiC. The plasmid pTrcBubiC was introduced into the strain CFT5 produced in Example 1 to produce a strain CFT54.

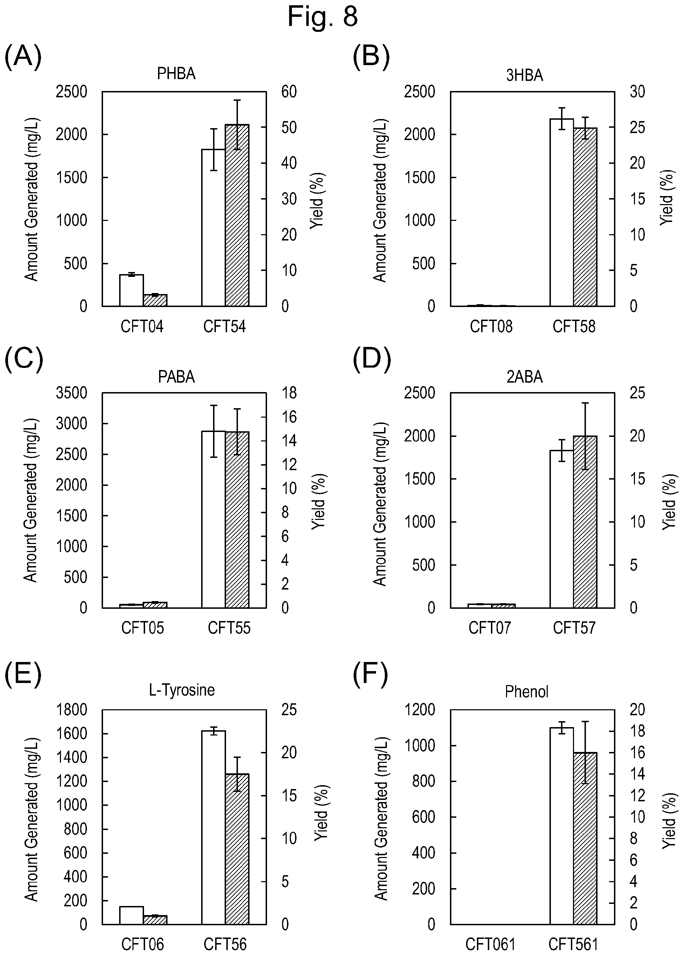

Figure 8(A) illustrates the amount and the yield of PHBA generated in a culture of the strain CFT54. The amount of PHBA generated in the strain CFT54 was as large as 1,820 mg/L after the culture for 96 hours. The yield of PHBA was increased by 15.8 times as compared with that in a strain CFT04, that is, a control strain obtained by introducing pTrcBubiC into the strain ATCC31882.

### [Example 7] Generation Promotion of 3-Hydroxybenzoic Acid

In the same manner as in Example 6, generation of a structural isomer of salicylic acid, 3-hydroxybenzoic acid (3HBA), was examined. In order to enable 3HBA to be generated from chorismic acid, a gene (SEQ ID NO:40, Hyg5) encoding 3-hydroxybenzoate synthase (EC 4.1.3.45) derived from *S. hygroscopicus* was introduced.

A gene fragment encoding hyg5 was amplified by PCR using a synthetic gene fragment as a template, and using hyg5_f (SEQ ID NO:19) and hyg5_r (SEQ ID NO:20) as primers. The resultant gene fragment was assembled together with a fragment of pTrcHis B (Life Technologies Corporation) using Gibson Assembly (New England BioLabs, Inc.). The obtained plasmid was designated as pTrcBhyg5. The plasmid pTrcBhyg5 was introduced into the strain CFT5 produced in Example 1 to produce a strain CFT58.

Figure 8(B) illustrates the amount and the yield of 3HBA generated in a culture of the strain CFT58. The amount of 3HBA generated in the strain CFT58 was as large as 2,180 mg/L after the culture for 72 hours. The yield of 3HBA was increased by 310 times as compared with that in a strain CFT08, that is a control strain obtained by introducing pTrcBhyg5 into the strain ATCC31882.

### [Example 8] Generation Promotion of 4-Aminobenzoic Acid

Generation of 4-aminobenzoic acid (PABA) was examined using the cell line of the present invention. In order to produce PABA from chorismic acid, aminodeoxychorismate synthase (EC 2.6.1.85) and 4-amino-4-deoxychorismate lyase (EC 4.1.3.38) (PabA, PabB and PabC) derived from *E. coli* were used.

Gene fragments encoding pabA and pabB were amplified by the PCR using the genomic DNA of *E. coli* MG1655 as a template, and using pabA_f (SEQ ID NO:11) and pabA_r (SEQ ID NO:12), and pabB_f (SEQ ID NO:13) and pabB_r (SEQ ID NO:14) as respective primers. The thus amplified fragments were tandemly inserted into the KpnI site of pZE12MCS (Expressys). The obtained plasmid was designated as pZE12pabAB. A gene fragment of pabC derived from *E. coli* was purchased from Invitrogen (SEQ ID NO:42). This gene fragment was directly inserted into the PstI site of pZE12pabAB. The obtained plasmid was designated as pZE12pabABC. The plasmid pZE12pabABC was introduced into the strain CFT5 produced in Example 1 to produce a strain CFT55.

Figure 8(C) illustrates the amount and the yield of PABA generated in a culture of the strain CFT55. The amount of PABA generated after the culture for 48 hours was 2,880 mg/L. The yield of PABA was increased by 32.1 times as compared with that in a strain CFT05, that is, a control strain obtained by introducing pZE12pabABC into the strain ATCC31882.

### [Example 9] Generation Promotion of 2-Aminobenzoic Acid

Generation of 2-aminobenzoic acid (2ABA) in the cell lines of the present invention was examined. In order to produce 2ABA from chorismic acid, a part of L-tryptophan biosynthetic pathway of *E. coli* can be used, and genes (trpE and trpG) encoding anthranilate synthase (EC 4.1.3.27) derived from *E. coli* were introduced.

A gene fragment encoding trpEG was amplified by the PCR using the genomic DNA of *E. coli* MG1655 as a template, and using trpEG_f (SEQ ID NO:15) and trpEG_r (SEQ ID NO:16) as primers. The amplified fragment was inserted into the KpnI site of the pZE12MCS (Expressys). The obtained plasmid was designated as pZE12trpEG. The plasmid pZE12trpEG was introduced into the strain CFT5 produced in Example 1 to produce a strain CFT57.

Figure 8(D) illustrates the amount and the yield of 2ABA generated in a culture of the strain CFT57. The amount of 2ABA generated after the culture for 48 hours was 1,830 mg/L. The yield of 2ABA was increased by 46.5 times as compared with that in a strain CFT07, that is a control strain obtained by introducing pZE12trpEG into the strain ATCC31882.

### [Example 10] Generation Promotion of L-tyrosine

The strain CFT5 was used for producing an *E. coli* strain capable of producing L-tyrosine. In order to produce L-tyrosine from chorismic acid, a gene (SEQ ID NO:43, tyrA^{fbr}) encoding chorismate mutase/prephenate dehydratase (EC 5.4.99.5 and EC 4.2.1.51) derived from *E. coli* was introduced.

As a gene fragment of tyrA^{fbr} derived from *E. coli,* a commercially available product was obtained (Invitrogen). The gene fragment was directly inserted into the KpnI site of the pZA23MCS (Expressys). The obtained plasmid was designated as pZA23tyrA^{fbr}. The plasmid pZA23tyrA^{fbr} was introduced into the strain CFT5 produced in Example 1 to produce a strain CFT56.

Figure 8(E) illustrates the amount and the yield of L-tyrosine generated in a culture of the strain CFT56. The amount of L-tyrosine generated was as large as 1,620 mg/L after the culture for 96 hours, and the yield was increased by 10.8 times as compared with that in a strain CFT06, that is a control strain obtained by introducing pZA23tyrA^{fbr} into the strain ATCC31882.

### [Example 11] Generation Promotion of Phenol

The L-tyrosine overproducing strain obtained in Example 10 was used for producing a strain producing phenol that can be synthesized from L-tyrosine by tyrosine phenol lyase (TPL) [EC 4.1.99.2].

A gene fragment encoding tpl was amplified by PCR using a gene fragment synthesized based on a gene sequence (SEQ ID NO:44, tpl) encoding TPL derived from *Pasteurella multocida* as a template, and using tpl_f (SEQ ID NO:17) and tpl_r (SEQ ID NO. 18) as primers. A gene fragment of pTrcHis B was also amplified by PCR using pTrcHis B (Life Technologies Corporation) as a template, and using pTrc_inv_f (SEQ ID NO:21) and pTrc_inv_r (SEQ ID NO:22) as primers. These fragments were assembled using Gibson Assembly (New England BioLabs, Inc.). The obtained plasmid was designated as pTrcBtpl. The plasmid pTrcBtpl was introduced into the strain CFT56 produced in Example 10 to produce a strain CFT561.

Figure 8(F) illustrates the amount and the yield of phenol generated in a culture of the strain CFT561. The amount of phenol generated was as large as 1,100 mg/L after the culture for 96 hours, and remarkably higher phenol generation ability than that of a strain CFT061, that is a control strain obtained by introducing pTrcBtpl into the strain CFT06, was exhibited.

### [Example 12] Generation Promotion of Muconic Acid

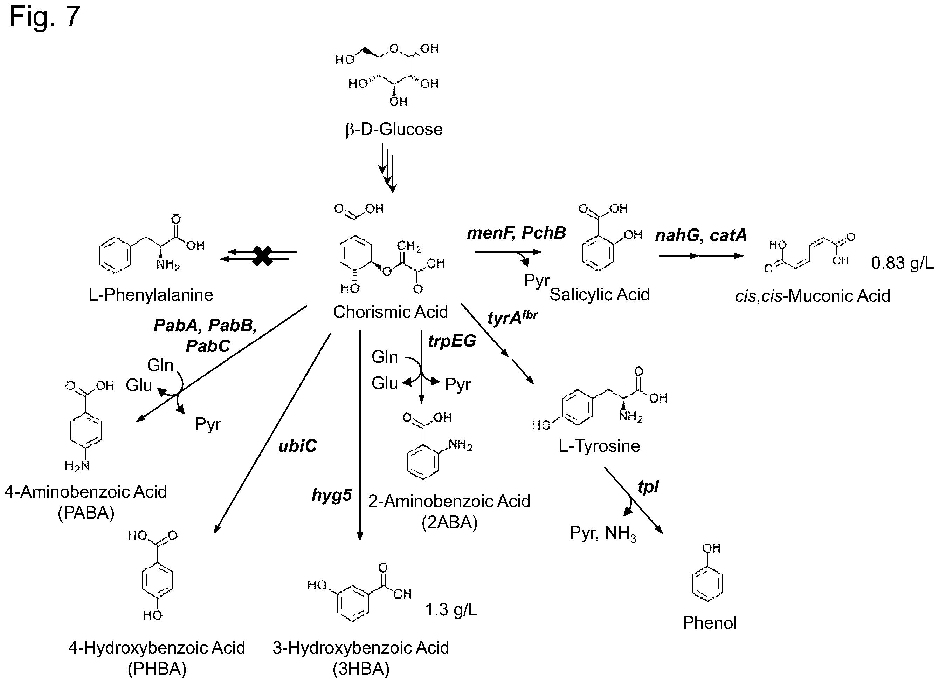

Muconic acid can be generated from salicylic acid through a reaction catalyzed by salicylate 1-monooxygenase (SMO) and catechol 1,2-dioxygenase (CDO) (Figure 7).

As gene fragments of SMO (SEQ ID NO:45, nahG^{opt}) derived from *Pseudomonas putida* KT2440 and CDO (SEQ ID NO:46, catA) derived from *Pseudomonas putida* DOT-TIE, commercially available products (Invitrogen) were used. The gene fragment of nahG^{opt} was directly inserted into the KpnI site of pZE12MCS (Expressys), and the resultant was designated as pZE12nahG. Subsequently, the gene fragment of catA was inserted into the HindIII site of pZE12nahG. The obtained plasmid was designated as pZE12nGcA. The plasmid pZE12nGcA was introduced, together with pZA23mI, into the strain CFT5 to produce a strain CFT591.

After the culture for 96 hours, the amount of muconic acid generated from glucose used as an only carbon source was as large as 830 mg/L in a culture of the strain CFT591, and remarkably higher muconic acid generation ability was exhibited, than that of a strain CFT091, that is a control strain obtained by introducing pZE12nGcA into the strain CFT09 produced in Example 2.

### [Example 13] Generation Promotion of Maleic Acid

The cell line of the present invention was used to produce a strain generating maleic acid from chorismic acid via 3-hydroxybenzoic acid, gentisic acid and maleylpyruvic acid (Figure 9).

Into the strain CFT5 produced in Example 1, a gene encoding 3-hydroxybenzoate synthase, a gene encoding 3-hydroxybenzoate 6-hydroxylase, a gene encoding gentisate-1,2-dioxygenase, and a gene encoding maleylpyruvate hydrolase were introduced.

As gene fragments of hyg5 (SEQ ID NO:40) derived from *S. hygroscopicus,* 3hb6h (SEQ ID NO:47) derived from *Rhodococcus jostii* RHA1, mps2 (SEQ ID NO:48) derived from *Rhodococcus sp.* NCIMB 12038, and hbzF (SEQ ID NO:49) derived from *Pseudomonas alcaligenes* NCIMB 9867, used in this example, commercially available products (Invitrogen) were used.

A gene fragment encoding 3H6H was amplified by PCR using the above-described 3hb6h gene fragment as a template, and using 3hb6h_f (SEQ ID NO:50) and 3hb6h_r (SEQ ID NO:51) as primers. Similarly, mps2 gene fragment and hbzF gene fragment were respectively used to amplify a gene fragment encoding GDO and a gene fragment encoding MPH.

The amplified mps2, hbzF, hyg5 and 3hb6h gene fragments were tandemly inserted into pTrcHisB (Life Technologies Corporation), and the obtained plasmid was designated as pT2t01. The plasmid pT2t01 was introduced into the strain CFT5 produced in Example 1 to produce a strain CFMt1. As a result, although the generation of maleic acid was not found in the strain CFT5 without introducing the gene, the amount of maleic acid generated in the strain CFMt1 was as large as 1,210 mg/L after the culture for 144 hours, and it was confirmed that the amount of maleic acid generated was remarkably increased.

Next, in order to obtain a cell line having further improved maleic acid generation ability, further gene introduction was examined. Two plasmids pS09 and pA09 each containing the hyg5 gene fragment together with a Ptrc promoter, and a plasmid pS10 containing the hyg5 and 3hb6h gene fragments together with a Ptrc promoter were respectively produced (see Table 2). Each of the plasmids pS09 and pS10 was introduced into the above-described strain CFMt1 to produce strains CMtS09 and CMtS10, respectively. Further, the plasmid pA09 was introduced into each of the strains CMtS09 and CMtS10 to produce strains CMtS091 and CMtS101 (see Table 1).

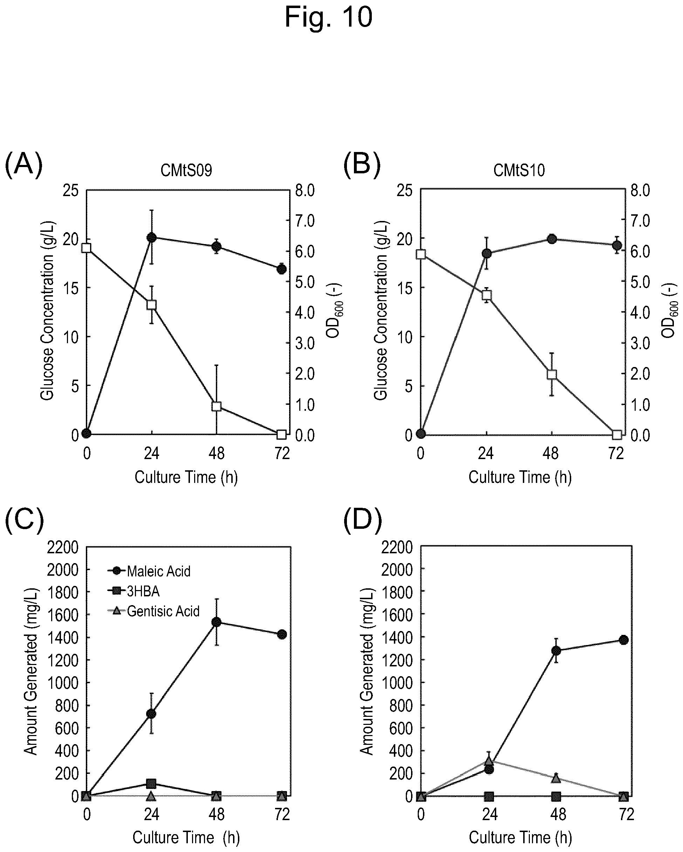

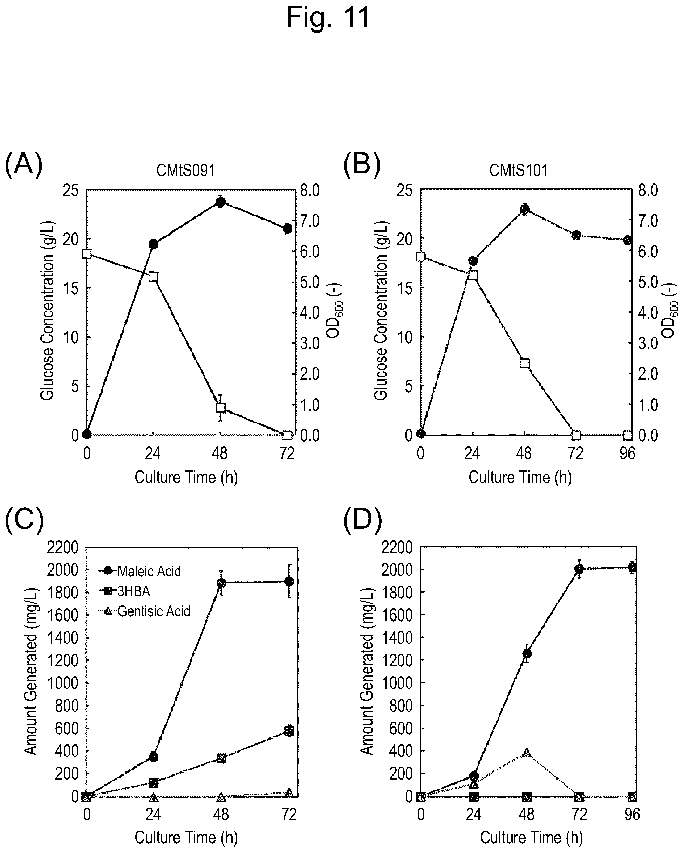

Figure 10 illustrates the amounts of maleic acid generated in cultures of the strains CMtS09 and CMtS10, and Figure 11 illustrates the amounts of maleic acid generated in cultures of the strains CMtS091 and CMtS101. As illustrated, these cell lines have much higher maleic acid generation ability than the strain CFMt1, and the amount of maleic acid generated was as large as 2,000 mg/L after the culture of the strain CFMtS101 for 72 hours (Figure 11(D)). Although generation of 3-hydroxybenzoic acid and gentisic acid was also found in these cell lines, generation of maleylpyruvic acid was little, suggesting rapid conversion into maleic acid in the cell lines used.

### Industrial Applicability

The present invention provides various microorganisms having sugar metabolic pathways modified. By increasing the availability of PEP in a cell of microorganisms and blocking the flow of a carbon source to L-phenylalanine and L-tyrosine, the amount and the yield of salicylic acid generated from a carbon source such as glucose can be largely increased. Besides, a microorganism of the present invention can be used to produce various aromatic compounds derived from chorismic acid. The present invention can also provide a platform cell line to be used for synthesizing an aromatic compound. According to the present invention, various aromatic compounds can be obtained with high productivity using microorganisms with glucose, that is a major component of biomass, used as a carbon source.

All the publications, patents and patent applications cited herein are hereby incorporated by reference.

## Claims

1. A method for producing a microorganism having a sugar metabolic pathway modified, comprising:
suppressing the expression of a gene encoding a phosphotransferase system enzyme of the microorganism;
suppressing the expression of a gene encoding pyruvate kinase of the microorganism; and
introducing, into the microorganism, one or more genes encoding an enzyme that enables the microorganism to synthesize an aromatic compound from chorismic acid or isochorismic acid.

2. The method according to claim 1, further comprising suppressing the expression of a gene encoding an enzyme synthesizing L-phenylalanine from chorismic acid.

3. The method according to claim 1 or 2, wherein the aromatic compound is salicylic acid, and a gene encoding isochorismate synthase and a gene encoding isochorismate pyruvate lyase are introduced.

4. The method according to claim 1 or 2, wherein the aromatic compound is 4-hydroxybenzoic acid, and a gene encoding chorismate pyruvate lyase is introduced.

5. The method according to claim 1 or 2, wherein the aromatic compound is 3-hydroxybenzoic acid, and a gene encoding 3-hydroxybenzoate synthase is introduced.

6. The method according to claim 1 or 2, wherein the aromatic compound is 4-aminobenzoic acid, and a gene encoding aminodeoxychorismate synthase and a gene encoding 4-amino-4-deoxychorismate lyase are introduced.

7. The method according to claim 1 or 2, wherein the aromatic compound is 2-aminobenzoic acid, and a gene encoding anthranilate synthase is introduced.

8. The method according to claim 1 or 2, wherein the aromatic compound is L-tyrosine, and a gene encoding chorismate mutase and a gene encoding prephenate dehydratase are introduced.

9. The method according to claim 1 or 2, wherein the aromatic compound is phenol, and a gene encoding tyrosine phenol lyase is introduced.

10. The method according to claim 3, wherein a gene encoding salicylate 1-monooxygenase and a gene encoding catechol 1,2-dioxygenase are further introduced.

11. The method according to claim 5, wherein one or more genes that enable to synthesize gentisic acid, maleylpyruvic acid and/or maleic acid from the 3-hydroxybenzoic acid are further introduced.

12. A microorganism having a sugar metabolic pathway modified obtained by the method according to any one of claims 1 to 11.

13. The microorganism according to claim 12, wherein the microorganism is selected from bacteria, yeasts and fungi.

14. A method for producing an aromatic compound or a derivative thereof, comprising: culturing the microorganism according to claim 12 or 13; and recovering an aromatic compound or a derivative thereof from the culture.

15. The method according to claim 14, wherein the aromatic compound is salicylic acid, 4-hydroxybenzoic acid, 3-hydroxybenzoic acid, 4-aminobenzoic acid, 2-aminobenzoic acid, L-tyrosine or phenol, and the derivative is muconic acid, gentisic acid, maleylpyruvic acid or maleic acid.

16. The method according to claim 14 or 15, wherein only glucose is added as a carbon source.
